# EUROPÄISCHE PATENTSCHRIFT

(11) **EP 0 813 528 B1**
(45) Veröffentlichungstag und Bekanntmachung des Hinweises auf die Patenterteilung: **19.06.2002**
(21) Anmeldenummer: 96905829.6
(22) Anmeldetag: 01.03.1996
(51) Int. Cl.: C07D 249/12, A01N 47/38, C07D 401/12, C07D 403/12, C07D 405/12, C07D 409/12, C07D 417/12

(54) **HERBIZIDE ODER FUNGIZIDE SULFONYLAMINOCARBONYLTRIAZOLINONE MIT HALOGENALK(EN)OXY SUBSTITUENTEN**
HERBICIDAL OR FUNGICIDAL SULPHONYLAMINOCARBONYLTRIAZOLINONES WITH HALOGENATED ALK(EN)OXY SUBSTITUENTS
SULFONYLAMINOCARBONYLTRIAZOLINONES HERBICIDES OU FONGICIDES A SUBSTITUANTS ALC(EN)OXY HALOGENES

(30) Priorität: 08.03.1995 DE 19508118
(43) Veröffentlichungstag der Anmeldung: 29.12.1997
(73) Patentinhaber: BAYER AG, 51368 Leverkusen (DE)
(72) Erfinder: MÜLLER, Klaus-Helmut, D-40593 Düsseldorf (DE); KIRSTEN, Rolf, D-40789 Monheim (DE); GESING, Ernst R. F., D-40699 Erkrath-Hochdahl (DE); KLUTH, Joachim, D-40764 Langenfeld (DE); FINDEISEN, Kurt, D-51375 Leverkusen (DE); JANSEN, Johannes R., D-40789 Monheim (DE); KÖNIG, Klaus, D-51519 Odenthal (DE); RIEBEL, Hans-Jochem, D-42113 Wuppertal (DE); SCHALLNER, Otto, D-40789 Monheim (DE); WROBLOWSKY, Heinz-Jürgen, D-40764 Langenfeld (DE); DOLLINGER, Markus, D-51381 Leverkusen (DE); SANTEL, Hans-Joachim, D-51371 Leverkusen (DE); STENZEL, Klaus, D-40595 Düsseldorf (DE)
(86) Internationale Anmeldenummer: EP9600833
(87) Internationale Veröffentlichungsnummer: WO9627590

(56) Entgegenhaltungen:
- EP-A- 0 341 489
- EP-A- 0 477 646
- EP-A- 0 507 171
- EP-A- 0 534 266

## Beschreibung

Die Erfindung betrifft neue Sulfonylaminocarbonyltriazolinone mit Halogenalkoxy-Substituenten, mehrere Verfahren und neue Zwischenprodukte zu ihrer Herstellung sowie ihre Verwendung als Herbizide und Fungizide.

Es ist bereits bekannt, daß bestimmte Sulfonylaminocarbonyltriazolinone herbizide Eigenschaften aufweisen (vgl. EP-A 34148, EP-A 422469, EP-A 425948, EP-A 431291, EP-A 507171). Die Wirkung dieser Verbindungen ist jedoch nicht in allen Belangen zufriedenstellend.

Es wurden nun die neuen Sulfonylaminocarbonyltriazolinone mit Halogenalkoxy-Substituenten der allgemeinen Formel (I), 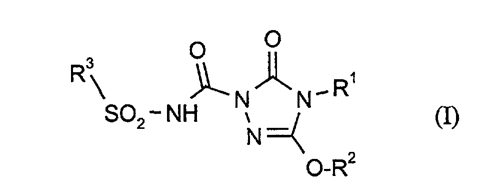 in welcher
- R¹: für Wasserstoff, Hydroxy, Amino, C₁-C₆-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor und/oder Chlor substituiertes C₁-C₆-Alkoxy oder C₂-C₆-Alkenyloxy, für jeweils gegebenenfalls durch Fluor und/oder Chlor substituiertes C₁-C₆-Alkylamino, Di-(C₁-C₄-alkyl)-amino oder C₁-C₄-Alkanoylamino, für jeweils gegebenenfalls durch Fluor, Chlor, Brom und/oder C₁-C₄-Alkyl substituiertes C₃-C₆-Cycloalkyl oder C₃-C₆-Cycloalkyl-C₁-C₄-alkyl, oder für jeweils gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl oder Phenyl-C₁-C₄-alkyl steht,
- R²: für -CH₂-CF₃, -CH₂-CCl₃ oder -CH₂-CF₂-CHF₂ steht und
- R³: für die Gruppierung 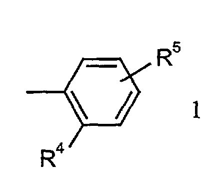 steht,
worin
R⁴ und R⁵ gleich oder verschieden sind und für Wasserstoff, Fluor, Chlor, Brom, Iod, Nitro, C₁-C₆-Alkyl (welches gegebenenfalls durch Fluor, Chlor, Brom, Cyano, Carboxy, C₁-C₄-Alkoxycarbonyl, C₁-C₄-Alkylamino-carbonyl, Di-(C₁-C₄-alkyl)amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfmyl, C₁-C₄-Alkylsulfonyl, Di-(C₁-C₄-alkyl)-aminosulfonyl, C₃-C₆-Cycloalkyl oder Phenyl substituiert ist), für C₂-C₆-Alkenyl (welches gegebenenfalls durch Fluor, Chlor, Brom, Cyano, C₁-C₄-Alkoxy-carbonyl, Carboxy oder Phenyl substituiert ist), für C₂-C₆-Alkinyl (welches gegebenenfalls durch Fluor, Chlor, Brom, Cyano, C₁-C₄-Alkoxy-carbonyl, Carboxy oder Phenyl substituiert ist), für C₁-C₄-Alkoxy (welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist), für C₁-C₄-Alkylthio (welches gegebenenfalls durch Fluor, Chlor, Brom, Cyano, Carboxy, C₁-C₄-Alkoxy-carbonyl, C₁-C₄-Alkylthio, C₁-C₄-Alkylsulfinyl oder C₁-C₄-Alkylsulfonyl substituiert ist), für C₂-C₆-Alkenyloxy (welches gegebenenfalls durch Fluor, Chlor, Brom, Cyano oder C₁-C₄-Alkoxy-carbonyl substituiert ist), für C₂-C₆-Alkenylthio (welches gegebenenfalls durch Fluor, Chlor, Brom, Cyano, Nitro, C₁-C₃-Alkylthio oder C₁-C₄-Alkoxycarbonyl substituiert ist), C₃-C₆-Alkinyloxy, C₃-C₆-Alkinylthio oder für den Rest -S(O)ₚ-R⁶ stehen, wobei
p für die Zahlen 1 oder 2 steht und
R⁶ für C₁-C₄-Alkyl (welches gegebenenfalls durch Fluor, Chlor, Brom, Cyano oder C₁-C₄-Alkoxy-carbonyl substituiert ist), C₃-C₆-Alkenyl, C₃-C₆-Alkinyl, C₁-C₄-Alkoxy, C₁-C₄-Alkoxy-C₁-C₄-alkylamino, C₁-C₄-Alkylamino, Di-(C₁-C₄-alkyl)-amino, Phenyl oder für den Rest -NHOR⁷ steht, wobei
R⁷ für C₁-C₁₂-Alkyl (welches gegebenenfalls durch Fluor, Chlor, Cyano, C₁-C₄-Alkoxy, C₁-C₄-Alkylthio, C₁-C₄-Alkylsulfinyl, C₁-C₄-Alkylsulfonyl, C₁-C₄-Alkyl-carbonyl, C₁-C₄-Alkoxy- carbonyl, C₁-C₄-Alkylamino-carbonyl oder Di-(C₁-C₄-alkyl)-amino-carbonyl substituiert ist), für C₃-C₆-Alkenyl (welches gegebenenfalls durch Fluor, Chlor oder Brom substituiert ist), C₃-C₆-Alkinyl, C₃-C₆-Cycloalkyl, C₃-C₆-cycloalkyl-C₁-C₂-alkyl, Phenyl-C₁-C₂-alkyl (welches gegebenenfalls durch Fluor, Chlor, Nitro, Cyano, C₁-C₄-Alkyl, C₁-C₄-Alkoxy oder C₁-C₄-Alkoxy-carbonyl substituiert ist), für Benzhydryl oder für Phenyl (welches gegebenenfalls durch Fluor, Chlor, Nitro, Cyano, C₁-C₄-Alkyl, Trifluormethyl, C₁-C₄-Alkoxy, C₁-C₂-Fluoralkoxy, C₁-C₄-Alkylthio, Trifluormethylthio oder C₁-C₄-Alkoxycarbonyl substituiert ist) steht,
R⁴ und/oder R⁵ weiterhin für Phenyl oder Phenoxy, für C₁-C₄-Alkylcarbonylamino, C₁-C₄-Alkoxy-carbonylamino, C₁-C₄-Alkylamino-carbonyl-amino, Di-(C₁-C₄-alkyl)-amino-carbonylamino, oder für den Rest -CO-R⁸ stehen, wobei
R⁸ für Wasserstoff, C₁-C₆-Alkyl, C₃-C₆-Cycloalkyl, C₁-C₆-Alkoxy, C₃-C₆-Cycloalkoxy, C₃-C₆-Alkenyloxy, C₃-C₆-Alkinyloxy, C₁-C₄-Alkylthio, C₁-C₄-Alkylamino, C₁-C₄-Alkoxyamino, C₁-C₄-Alkoxy-C₁-C₄alkyl-amino oder Di-(C₁-C₄-alkyl)-amino steht (welche gegebenenfalls durch Fluor und/oder Chlor substituiert sind),
- R⁴ und/oder R⁵: weiterhin für Trimethylsilyl, Thiazolinyl, C₁-C₄-Alkylsulfonyloxy, Di-(C₁-C₄-alkyl)-aminosulfonylamino oder für den Rest
-CH=N-R⁹ stehen, wobei
R⁹ für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/oder Chlor substituiertes C₁-C₆-Alkoxy, C₃-C₆-Alkenoxy, C₃-C₆-Alkinoxy oder Benzyloxy, für Amino, C₁-C₄-Alkylamino, Di-(C₁-C₄-alkyl)-amino, Phenylamino, C₁-C₄-Alkyl-carbonyl-amino, C₁-C₄-Alkoxy-carbonylamino, C₁-C₄-Alkyl-sulfonylamino oder für gegebenenfalls durch Fluor, Chlor, Brom oder Methyl substituiertes Phenylsulfonylamino steht, weiterhin
- R³: für den Rest 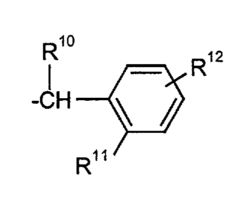 steht,
worin
- R¹⁰: für Wasserstoff oder C₁-C₄-Alkyl steht,
- R¹¹ und R¹²: gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Carboxy, C₁-C₄-Alkoxy-carbonyl, Dimethylaminocarbonyl, C₁-C₄-Alkylsulfonyl oder Di-(C₁-C₄-alkyl)-aminosulfonyl stehen;
weiterhin
- R³: für den Rest 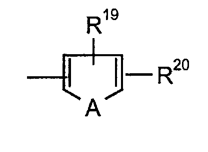 steht,
worin
- R¹⁹ und R²⁰: gleich oder verschieden sind und für Wasserstoff, Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), C₁-C₄-Alkylthio, C₁-C₄-Alkylsulfinyl oder C₁-C₄-Alkylsulfonyl (welches gegebenenfalls durch Fluor und/oder Chlor substituiert ist), Di-(C₁-C₄-alkyl)-amino-sulfonyl, C₁-C₄-Alkoxy-carbonyl oder Dimethylaminocarbonyl stehen, und
- A: für Schwefel steht.

Man erhält die neuen Sulfonylaminocarbonyltriazolinone mit Halogenalkoxy-Substituenten der allgemeinen Formel (I), wenn man
(a) Triazolinone der allgemeinen Formel (II) 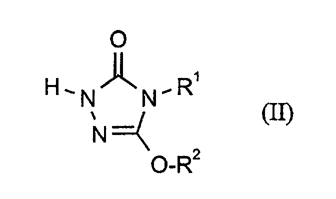 in welcher
   - R¹ und R²: die oben angegebene Bedeutung haben,
   mit Sulfonylisocyanaten der allgemeinen Formel (III)

   R³-SO₂-N=C=O (III)

   in welcher
   - R³: die oben angegebene Bedeutung hat,
   gegebenenfalls in Gegenwart eines Reaktionshilfsmittels und gegebenenfalls in Gegenwart eines Verdünnungsmittels umsetzt,
   oder wenn man
(b) Triazolinon-Derivate der allgemeinen Formel (IV) 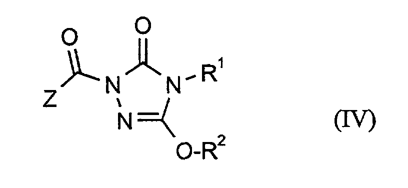 in welcher
   - R¹ und R²: die oben angegebene Bedeutung haben und
   - Z: für Halogen, Alkoxy, Aralkoxy oder Aryloxy steht,
   mit Sulfonsäureamiden der allgemeinen Formel (V)

   R³-SO₂-NH₂ (V)

   in welcher
   - R³: die oben angegebene Bedeutung hat,
   gegebenenfalls in Gegenwart eines Säureakzeptors und gegebenenfalls in Gegenwart eines Verdünnungsmittels umsetzt,
   oder wenn man
(c) Triazolinone der allgemeinen Formel (II) 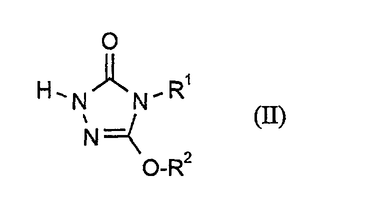 in welcher
   - R¹ und R²: die oben angegebene Bedeutung haben,
   mit Sulfonsäureamid-Derivaten der allgemeinen Formel (VI)

   R³-SO₂-NH-CO-Z (VI)

   in welcher
   - R³: die oben angegebene Bedeutung hat und
   - Z: für Halogen, Alkoxy, Aralkoxy oder Aryloxy steht,
   gegebenenfalls in Gegenwart eines Säureakzeptors und gegebenenfalls in Gegenwart eines Verdünnungsmittels umsetzt,
   oder wenn man
(d) Triazolinone der allgemeinen Formel (II) 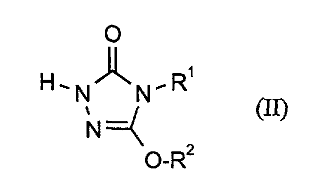
in welcher
- R¹ und R²: die oben angegebene Bedeutung haben,
mit Sulfonsäurehalogeniden der allgemeinen Formel (VII)

R³-SO₂-X (VII)

in welcher
R³ die oben angegebene Bedeutung hat und
X für Halogen steht, und Metallcyanaten der allgemeinen Formel (VIII)

MOCN (VIII)

in welcher
M für ein Alkalimetall oder Erdalkalimetall-äquivalent steht, gegebenenfalls in Gegenwart eines Reaktionshilfsmittels und gegebenenfalls in Gegenwart eines Verdünnungsmittels umsetzt,
und gegebenenfalls die nach Verfahren (a), (b), (c) oder (d) erhaltenen Verbindungen der Formel (I) nach üblichen Methoden in Salze überführt.

Die neuen Sulfonylaminocarbonyltriazolinone mit Halogenalkoxy-Substituenten der allgemeinen Formel (I) zeichnen sich durch starke herbizide und/oder fungizide Wirksamkeit aus.

Gegenstand der Erfindung sind weiter vorzugsweise Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium, Tri-(C₁-C₄alkyl)-sulfonium-, C₅- oder C₆-Cycloalkyl-ammonium- und Di-(C₁-C₂-alkyl)-benzylammonium-Salze von Verbindungen der Formel (I), in welcher n, R¹, R² und R³ die oben vorzugsweise angegebenen Bedeutungen haben.

Gegenstand der Erfindung sind insbesondere Verbindungen der Formel (I), in welcher
- R¹: für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für jeweils gegebenenfalls durch Fluor und/oder Chlor substituiertes Methoxy, Ethoxy, n- oder i-Propoxy, n-, i-, s-oder t-Butoxy, Propenyloxy oder Butenyloxy, für Methylamino, Ethylamino, n- oder i-Propylamino, n-, i-, s- oder t-Butylamino, Dimethylamino oder Diethylamino, für jeweils gegebenenfalls durch Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Fluor, Chlor, Brom, Cyano, Methyl, Trifluormethyl oder Methoxy substituiertes Benzyl oder Phenyl steht,
- R²: für -CH₂-CF₃, -CH₂-CCl₃ oder -CH₂-CF₂-CHF₂ steht und
- R³: für den Rest 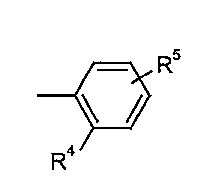 steht,
worin
- R⁴: für Fluor, Chlor, Brom, Methyl, Ethyl, n- oder i-Propyl, Trifluormethyl, Methoxy, Ethoxy, n- oder i-Propoxy, Difluormethoxy, Trifluormethoxy, 2-Chlor-ethoxy, 2-Methoxy-ethoxy, Methylthio, Ethylthio, n- oder i-Propylthio, Methylsulfinyl, Ethylsulfinyl, Methylsulfonyl, Ethylsulfonyl, Dimethylaminosulfonyl, Diethylaminosulfonyl, N-Methoxy-N-methylaminosulfonyl, Phenyl, Phenoxy, Methoxycarbonyl, Ethoxycarbonyl, n- oder i-Propoxycarbonyl, steht und
- R⁵: für Wasserstoff, Methyl, Ethyl, Fluor, Chlor oder Brom steht;
weiterhin
- R³: für den Rest 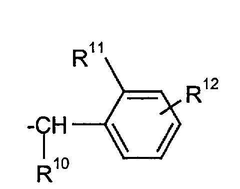 steht,
worin
- R¹⁰: für Wasserstoff steht,
- R¹¹: für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
- R¹²: für Wasserstoff steht;
weiterhin
- R³: für den Rest 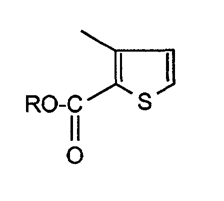 steht,
worin
- R: für Methyl, Ethyl, n- oder i-Propyl steht.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw.

Zwischenprodukte. Diese Restedefinitionen können untereinander, also auch zwischen den angegebenen Bereichen bevorzugter Verbindungen, beliebig kombiniert werden.

Die bei den Restedefinitionen genannten Kohlenwasserstoffreste, wie Alkyl, Alkenyl oder Alkinyl, auch in Kombinationen mit Heteroatomen, wie in Alkoxy, Alkylthio oder Alkylamino, sind auch dann, wenn dies nicht ausdrücklich angegeben ist, geradkettig oder verzweigt.

Halogen steht im allgemeinen für Fluor, Chlor, Brom oder Iod, vorzugsweise für Fluor, Chlor oder Brom, insbesondere für Fluor oder Chlor.

Verwendet man beispielsweise 2-Trifluormethoxy-phenylsulfonyl-isocyanat und 4-Ethyl-5-difluormethoxy-2,4-dihydro-3H-1,2,4-triazol-3-on als Ausgangsstoffe, so kann der Reaktionsablauf beim erfindungsgemäßen Verfahren (a) durch das folgende Formelschema skizziert werden:

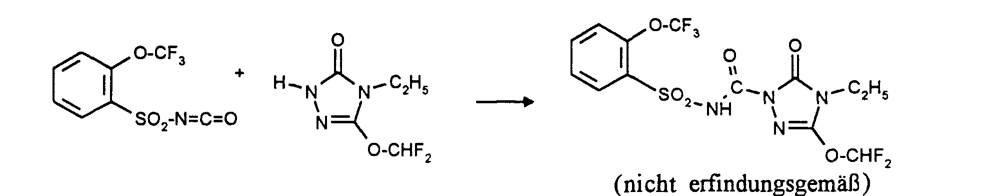

Verwendet man beispielsweise 2-Ethylthio-benzolsulfonamid und 2-Chlorcarbonyl-4-benzyl-5-(2,2-difluor-ethoxy)-2,4-dihydro-3H-1,2,4-triazol-3-on als Ausgangsstoffe, so kann der Reaktionsablauf beim erfindungsgemäßen Verfahren (b) durch das folgende Formelschema skizziert werden:

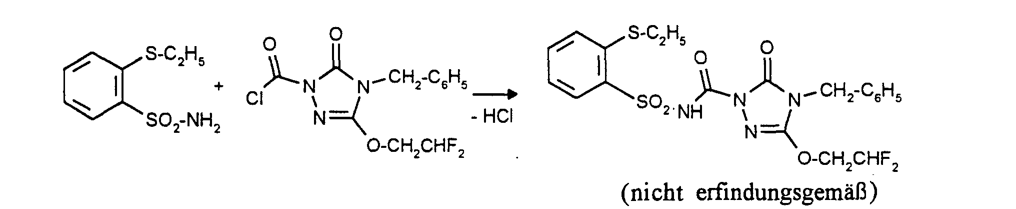

Verwendet man beispielsweise N-Methoxycarbonyl-2-methoxy-benzolsulfonamid und 5-(2-Chlor-ethoxy)-4-methyl-2,4-dihydro-3H-1,2,4-triazol-3-on als Ausgangsstoffe, so kann der Reaktionsablauf beim erfindungsgemäßen Verfahren (c) durch das folgende Formelschema skizziert werden:

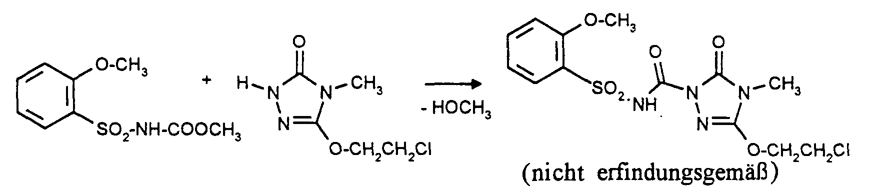

Verwendet man beispielsweise 2-Chlor-6-methyl-benzolsulfonsäurechlorid, 4-Allyl-5-(2,3,3-trifluor-propoxy)-2,4-dihydro-3H-1,2,4-triazol-3-on und Natriumcyanat als Ausgangsstoffe, so kann der Reaktionsablauf beim erfindungsgemäßen Verfahren (d) durch das folgende Formelschema skizziert werden:

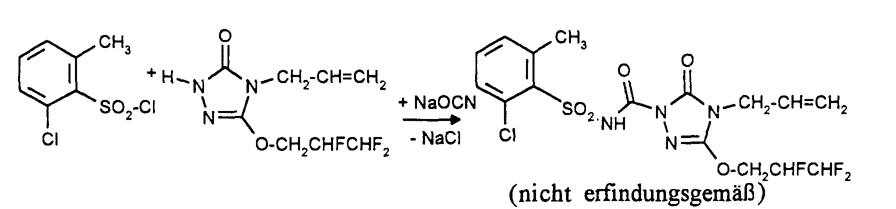

Die bei den erfindungsgemäßen Verfahren (a), (c) und (d) zur Herstellung von Verbindungen der Formel (I) als Ausgangsstoffe zu verwendenden Triazolinone sind durch die Formel (II) allgemein definiert.

In der Formel (II) haben R¹ und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ und R² angegeben wurden.

Die Triazolinone der allgemeinen Formel (II) sind noch nicht aus der Literatur bekannt; sie sind als neue Stoffe auch Gegenstand der vorliegenden Anmeldung.

Man erhält die neuen Triazolinone der Formel (II), wenn man Carbazinsäureester der allgemeinen Formel (IX) 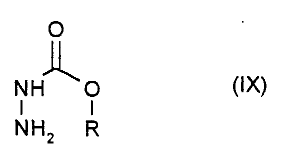 in welcher
- R: für Alkyl (vorzugsweise Methyl oder Ethyl) steht,
mit Alkyliminokohlensäure-diestern der allgemeinen Formel (X) 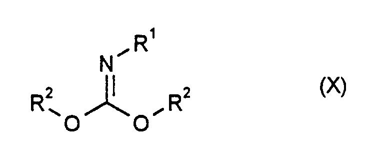 in welcher
- R¹ und R²: die oben angegebene Bedeutung haben,
gegebenenfalls in Gegenwart eines Verdünnungsmittels, wie z.B. Methanol, bei Temperaturen zwischen 0°C und 100°C umsetzt, die hierbei gebildeten Verbindungen der allgemeinen Formel (XI) 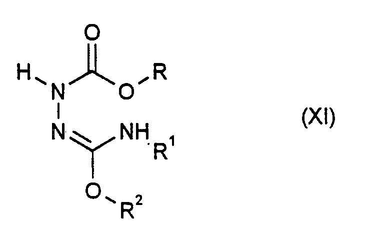 in welcher
- R, R¹ und R²: die oben angegebene Bedeutung haben,

- gegebenenfalls nach Zwischenisolierung - gegebenenfalls in Gegenwart eines Verdünnungsmittels, wie z.B. Toluol, bei Temperaturen zwischen 20°C und 150°C cyclisierend kondensiert (vgl. die Herstellungsbeispiele).

Die Triazolinone der Formel (II), bei welchen R² für Difluormethyl steht (wobei R¹ die oben angegebene Bedeutung hat), werden vorzugsweise durch Umsetzung von Urazolen der allgemeinen Formel (XII) 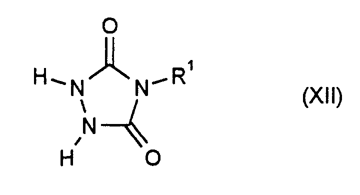 in welcher
- R¹: die oben angegebene Bedeutung hat,
mit Chlordifluormethan in Gegenwart eines Säureakzeptors, wie z.B. Natriumhydroxid, und in Gegenwart eines Verdünnungsmittels, wie z.B. Isopropanol und/oder Wasser, bei Temperaturen zwischen 0°C und 100°C hergestellt (vgl. die Herstellungsbeispiele).

Die beim erfindungsgemäßen Verfahren (a) zur Herstellung von Verbindungen der Formel (I) weiter als Ausgangsstoffe zu verwendenden Sulfonylisocyanate sind durch die Formel (III) allgemein definiert.

In der Formel (III) hat R³ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R³ angegeben wurde.

Die Ausgangsstoffe der Formel (III) sind bekannt und/oder können nach an sich bekannten Verfahren hergestellt werden (vgl. US-P 4127405, US-P 4169719, US-P 4371391, EP-A 7687, EP-A 13480, EP-A 21641, EP-A 23141, EP-A 23422, EP-A 30139, EP-A 35893, EP-A 44808, EP-A 44809, EP-A 48143, EP-A 51466, EP-A 64322, EP-A 70041, EP-A 173312).

Die beim erfindungsgemäßen Verfahren (b) zur Herstellung der Verbindungen der allgemeinen Formel (I) als Ausgangsstoffe zu verwendenden Triazolinon-Derivate sind durch die Formel (IV) allgemein definiert. In der Formel (IV) haben R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R¹ und R² angegeben wurden; Z steht vorzugsweise für Fluor, Chlor, Brom, Methoxy, Ethoxy, Benzyloxy, Phenoxy, Halogen- oder Nitro-phenoxy, insbesondere für Methoxy, Phenoxy oder 4-Nitrophenoxy.

Die Ausgangsstoffe der Formel (IV) sind noch nicht aus der Literatur bekannt; sie sind als neue Stoffe ebenfalls Gegenstand der vorliegenden Anmeldung.

Man erhält die neuen Verbindungen der Formel (IV), wenn man Triazolinone der allgemeinen Formel (II) 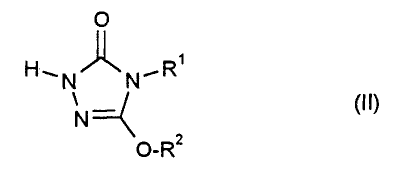 in welcher
- R¹ und R²: die oben angegebene Bedeutung haben,
mit Kohlensäurederivaten der allgemeinen Formel (XIII)

Z-CO-Z¹ (XIII)

in welcher
- Z: die oben angegebene Bedeutung hat und
- Z¹: für Halogen, Alkoxy, Aralkoxy oder Aryloxy steht,
gegebenenfalls in Gegenwart eines Säureakzeptors, wie z.B. Kalium-t-butylat, und gegebenenfalls in Gegenwart eines Verdünnungsmittels, wie z.B. Tetrahydrofuran oder Dimethoxyethan, bei Temperaturen zwischen 0°C und 100°C umsetzt.

Die beim erfindungsgemäßen Verfahren (b) zur Herstellung der Verbindungen der allgemeinen Formel (I) weiter als Ausgangsstoffe zu verwendenden Sulfonsäureamide sind durch die Formel (V) allgemein definiert. In der Formel (V) hat R³ vorzugsweise bzw. insbesondere diejenige Bedeutung, die bereits oben im Zusammenhang mit der Beschreibung der Verbindungen der Formel (I) als bevorzugt bzw. als insbesondere bevorzugt für R³ angegeben wurde.

Die Ausgangsstoffe der Formel (V) sind bekannt und/oder können nach an sich bekannten Verfahren hergestellt werden (vgl. US-P 4127405, US-P 4169719, US-P 4371391, EP-A 7687, EP-A 13480, EP-A 21641, EP-A 23141, EP-A 23422, EP-A 30139, EP-A 35893, EP-A 44808, EP-A 44809, EP-A 48143, EP-A 51466, EP-A 64322, EP-A 70041, EP-A 173312).

Die beim erfindungsgemäßen Verfahren (c) zur Herstellung der Verbindungen der Formel (I) als Ausgangsstoffe zu verwendenden Sulfonsäureamid-Derivate sind durch die Formel (VI) allgemein definiert. In der Formel (VI) hat R³ vorzugsweise bzw. insbesondere diejenige Bedeutung, die bereits oben im Zusammenhang mit der Beschreibung der Verbindungen der Formel (I) als bevorzugt bzw. als insbesondere bevorzugt für R³ angegeben wurde; Z steht vorzugsweise für Fluor, Chlor, Brom, Methoxy, Ethoxy, Benzyloxy oder Phenoxy, insbesondere für Methoxy oder Phenoxy.

Die Ausgangsstoffe der Formel (VI) sind bekannt und/oder können nach an sich bekannten Verfahren hergestellt werden.

Die beim erfindungsgemäßen Verfahren (d) zur Herstellung der Verbindungen der Formel (I) als Ausgangsstoffe zu verwendenden Sulfonsäurehalogenide sind durch die Formel (VII) allgemein definiert. In der Formel (VII) hat R³ vorzugsweise bzw. insbesondere diejenige Bedeutung, die bereits oben im Zusammenhang mit der Beschreibung der Verbindungen der Formel (I) als bevorzugt bzw. als insbesondere bevorzugt für R³ angegeben wurde; X steht vorzugsweise für Fluor, Chlor oder Brom, insbesondere für Chlor.

Die Ausgangsstoffe der Formel (VII) sind bekannt und/oder können nach an sich bekannten Verfahren hergestellt werden.

Die erfindungsgemäßen Verfahren (a), (b), (c) und (d) zur Herstellung der neuen Verbindungen der Formel (I) werden vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlormethan, Chlorbenzol und o-Dichlorbenzol; Ether wie Diethyl- und Dibutylether, Glykoldimethylether und Diglykoldimethylether, Tetrahydrofuran und Dioxan; Ketone wie Aceton, Methyl-ethyl-, Methyl-isopropyl- und Methyl-isobutyl-keton; Ester wie Essigssäuremethylester und -ethylester; Nitrile wie z.B. Acetonitril und Propionitril; Amide wie z.B. Dimethylformamid, Dimethylacetamid und N-Methylpyrrolidon sowie Dimethylsulfoxid, Tetramethylensulfon und Hexamethylphosphorsäuretriamid.

Als Reaktionshilfsmittel bzw. als Säureakzeptoren können bei den erfindungsgemäßen Verfahren (a), (b), (c) und (d) alle üblicherweise für derartige Umsetzungen verwendbaren Säurebindemittel eingesetzt werden. Vorzugsweise in Frage kommen Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kalium-tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO)

Die Reaktionstemperaturen können bei den erfindungsgemäßen Verfahren (a), (b), (c) und (d) in einem größeren Bereich variiert werden. Im allgemeinen arbeitet man bei Temperaturen zwischen -20°C und +100°C, vorzugsweise bei Temperaturen zwischen 0°C und +80°C.

Die erfindungsgemäßen Verfahren (a), (b), (c) und (d) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a), (b), (c) und (d) werden die jeweils benötigten Ausgangsstoffe im allgemeinen in angenähert äquimolaren Mengen eingesetzt. Es ist jedoch auch möglich, eine der jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a), (b), (c) und (d) jeweils nach üblichen Methoden (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfruchtund Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im NachauflaufVerfahren.

Die erfindungsgemäßen Wirkstoffe weisen zusätzlich auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Verbindungen der Formel (I) eignen sich insbesondere zur protektiven Behandlung von Kernobst, wie z.B. Äpfeln, gegen den Apfelmehltauerreger (Podosphaera leucotricha) und in gewissem Umfang auch zum Einsatz im Reis gegen Pyricularia oryzae.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 2 kg pro ha.

Die Herstellung und die Verwendung der erfindungsgemäßen Wirkstoffe geht aus den nachfolgenden Beispielen hervor.

### Herstellungsbeispiele:

### Beispiel 1

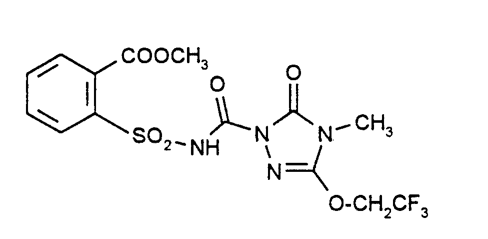

### (Verfahren (a))

Zu einer Lösung von 1,9 g (9,5 mMol) 4-Methyl-5-(2,2,2-trifluor-ethoxy)-2,4-dihydro-3H-1,2,4-triazol-3-on in 50 ml Acetonitril werden 2,4 g (10 mMol) 2-Methoxycarbonyl-phenylsulfonylisocyanat gegeben und die Mischung wird 12 Stunden bei 20°C gerührt. Dann wird im Vakuum eingeengt und der Rückstand mit Diethylether kristallisiert.

Man erhält 3,7 g (89% der Theorie) 2-(2-Methoxycarbonyl-phenylsulfonylaminocarbonyl)-4-methyl-5-(2,2,2-trifluor-ethoxy)-2,4-dihydro-3H-1,2,4-triazol-3-on vom Schmelzpunkt 153°C.

### Beispiel 2

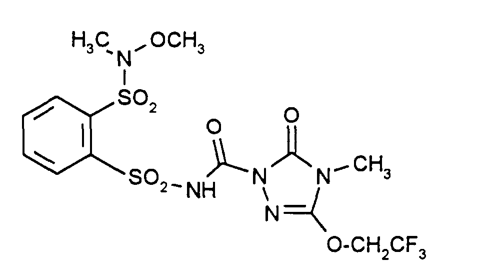

### (Verfahren (b))

3,8 g (12 mMol) 4-Methyl-2-phenoxycarbonyl-5-(2,2,2-trifluorethoxy)-2,4-dihydro-3H-1,2,4-triazol-3-on werden in 20 ml Acetonitril gelöst und mit 3,4 g (12 mMol) 2-(N-Methoxy-N-methyl-aminosulfonyl)-benzolsulfonamid und 1,9 g (12,5 mMol) Diazabicycloundecen (DBU) versetzt. Nach 1 Stunde Rühren bei 20°C wird auf eine Mischung aus Methylenchlorid/5%ige Salzsäure gegossen und die organische Phase abgetrennt. Nach Trocknen mit Natriumsulfat und Einengen im Vakuum wird der Rückstand mit Diethylether zur Kristallisation gebracht.

Man erhält 3,3 g (55% der Theorie) 2-[2-(N-Methoxy-N-methyl-aminosulfonyl)-phenylsulfonyl-aminocarbonyl]-4-methyl-5-(2,2,2-trifluorethoxy)-2,4-dihydro-3H-1,2,4-triazol-3-on vom Schmelzpunkt 183°C.

Analog zu den Beispielen 1 und 2 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

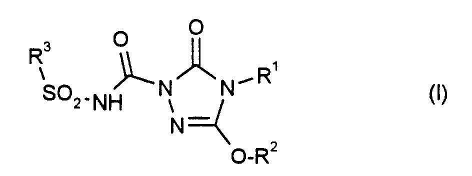

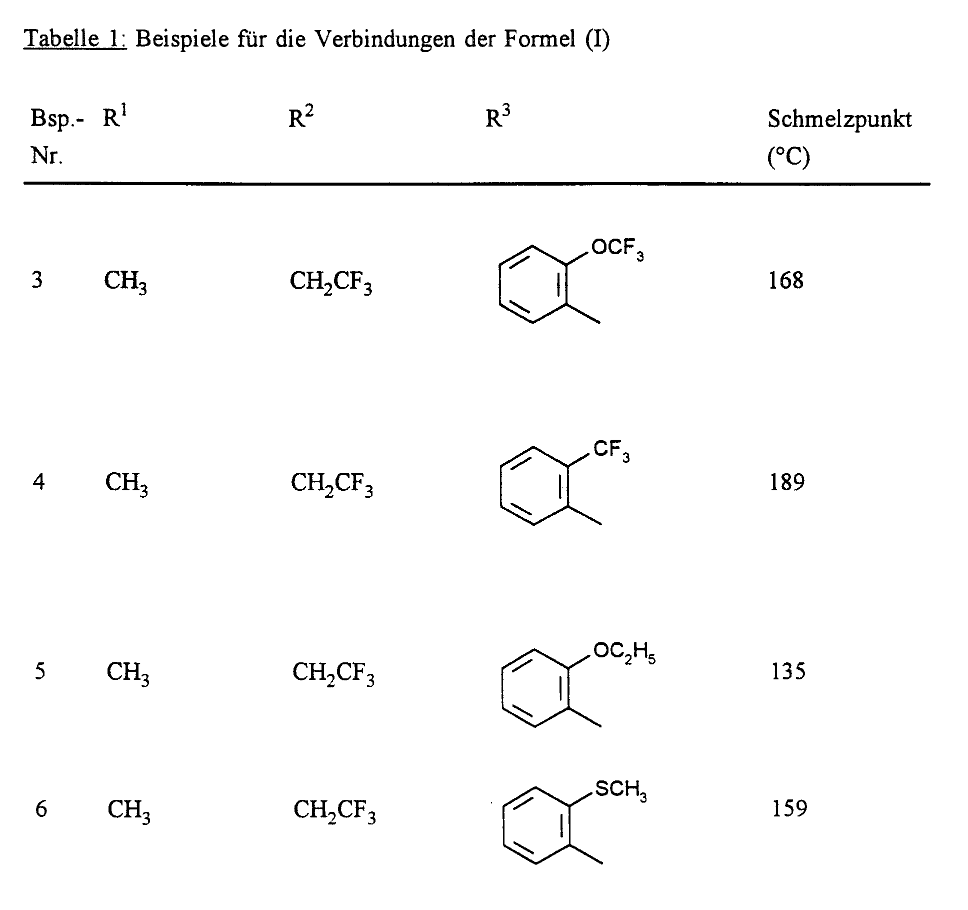

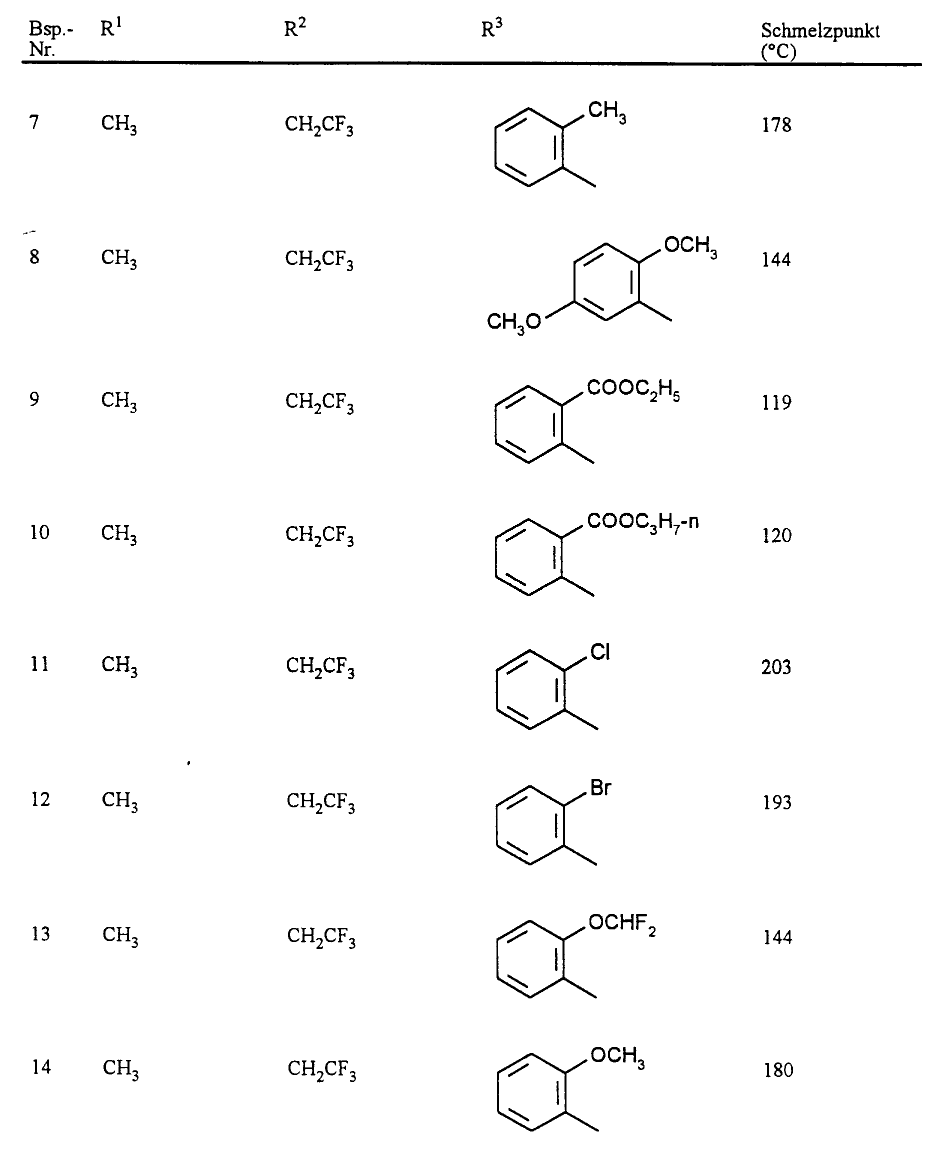

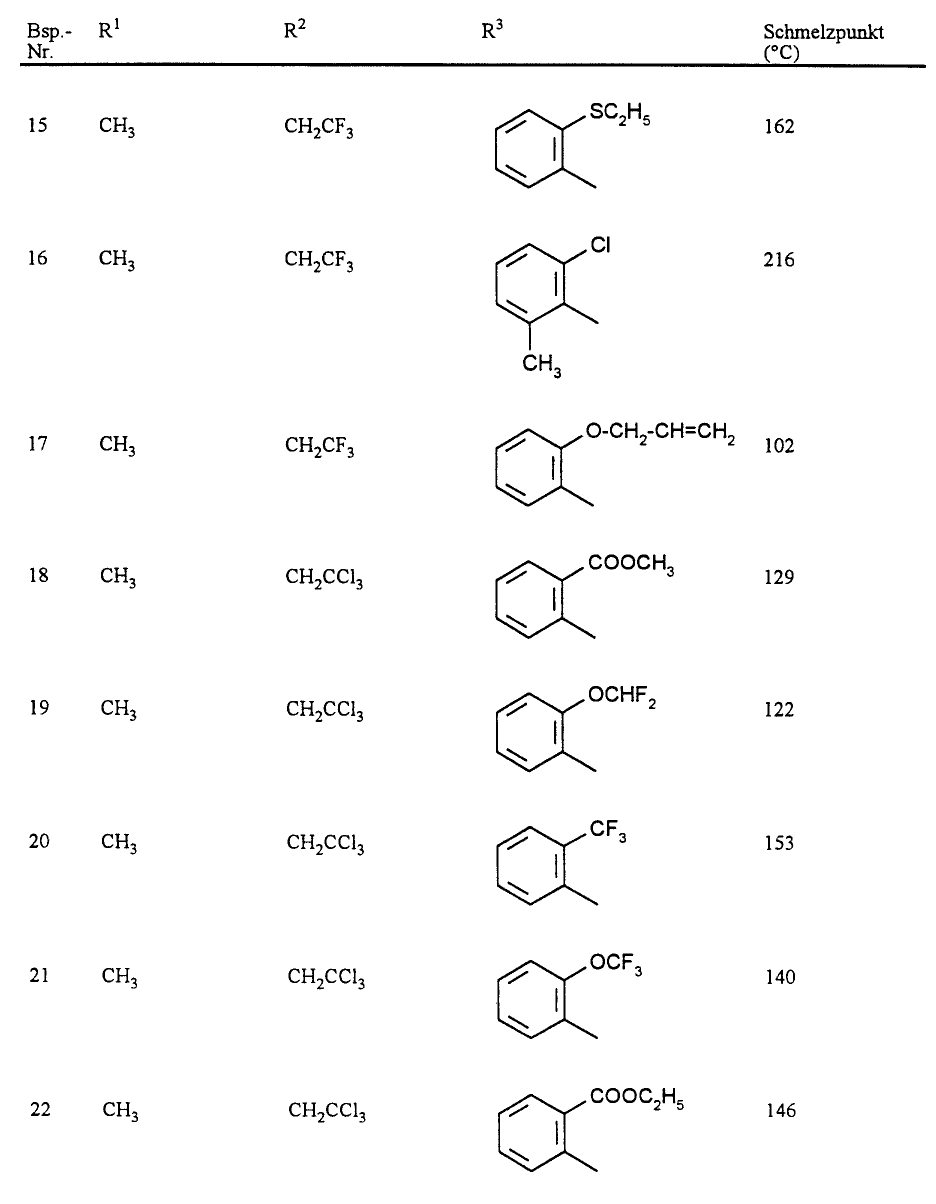

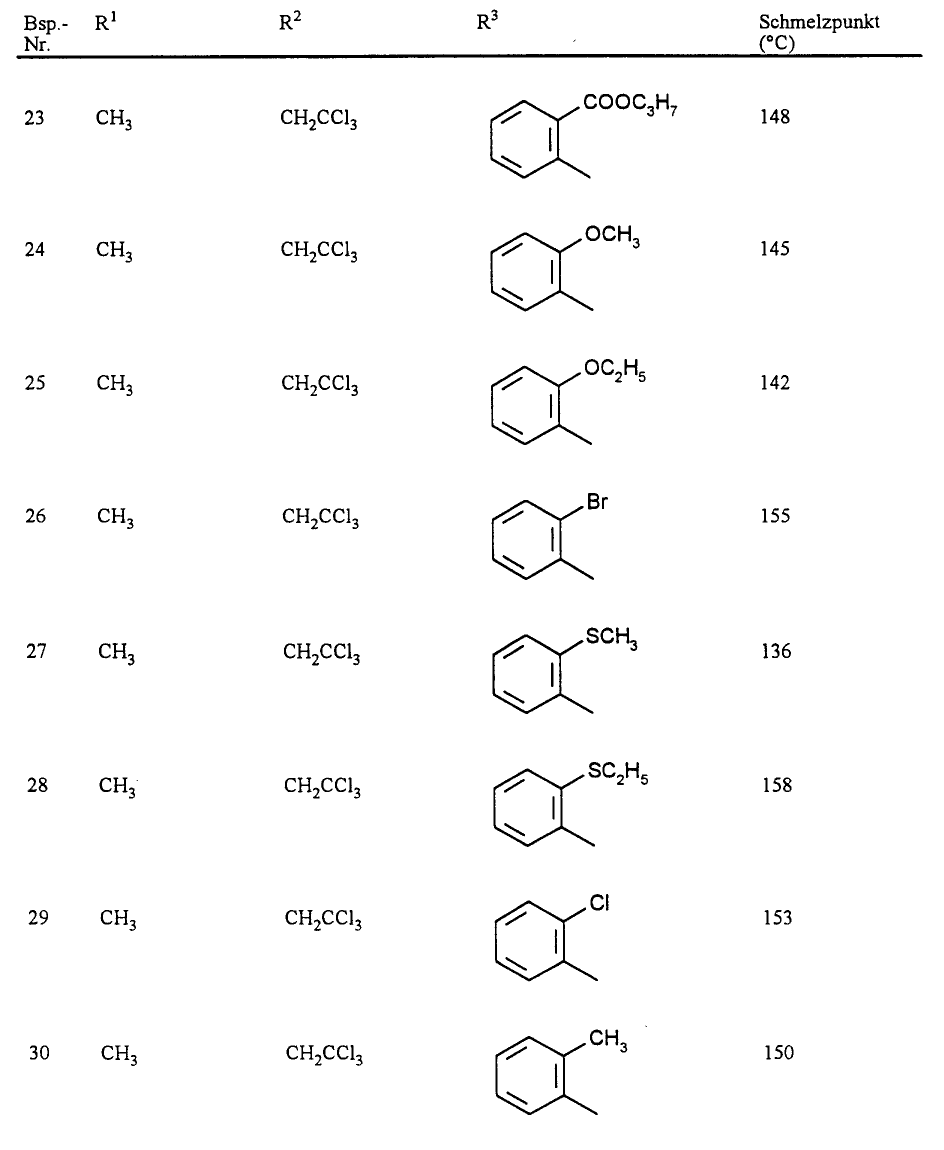

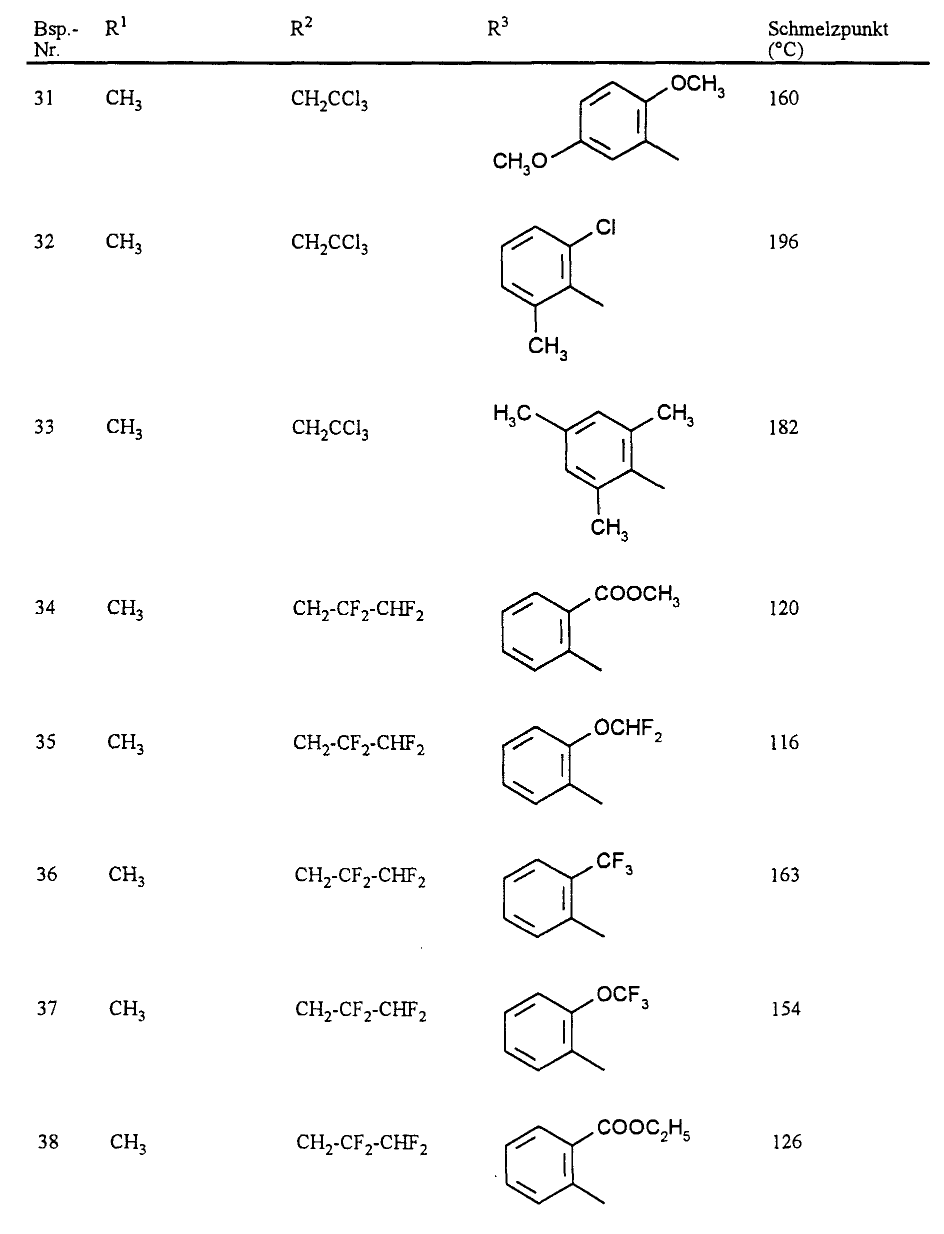

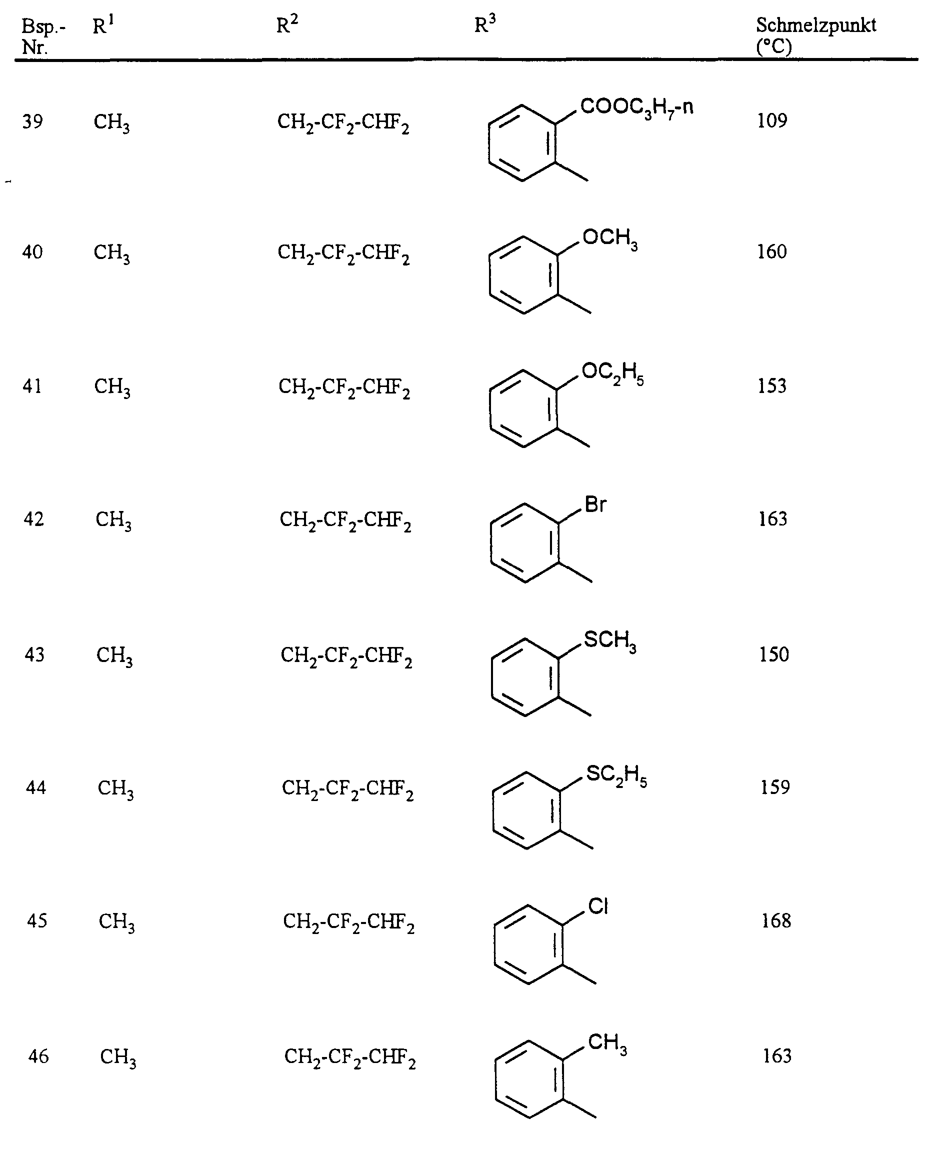

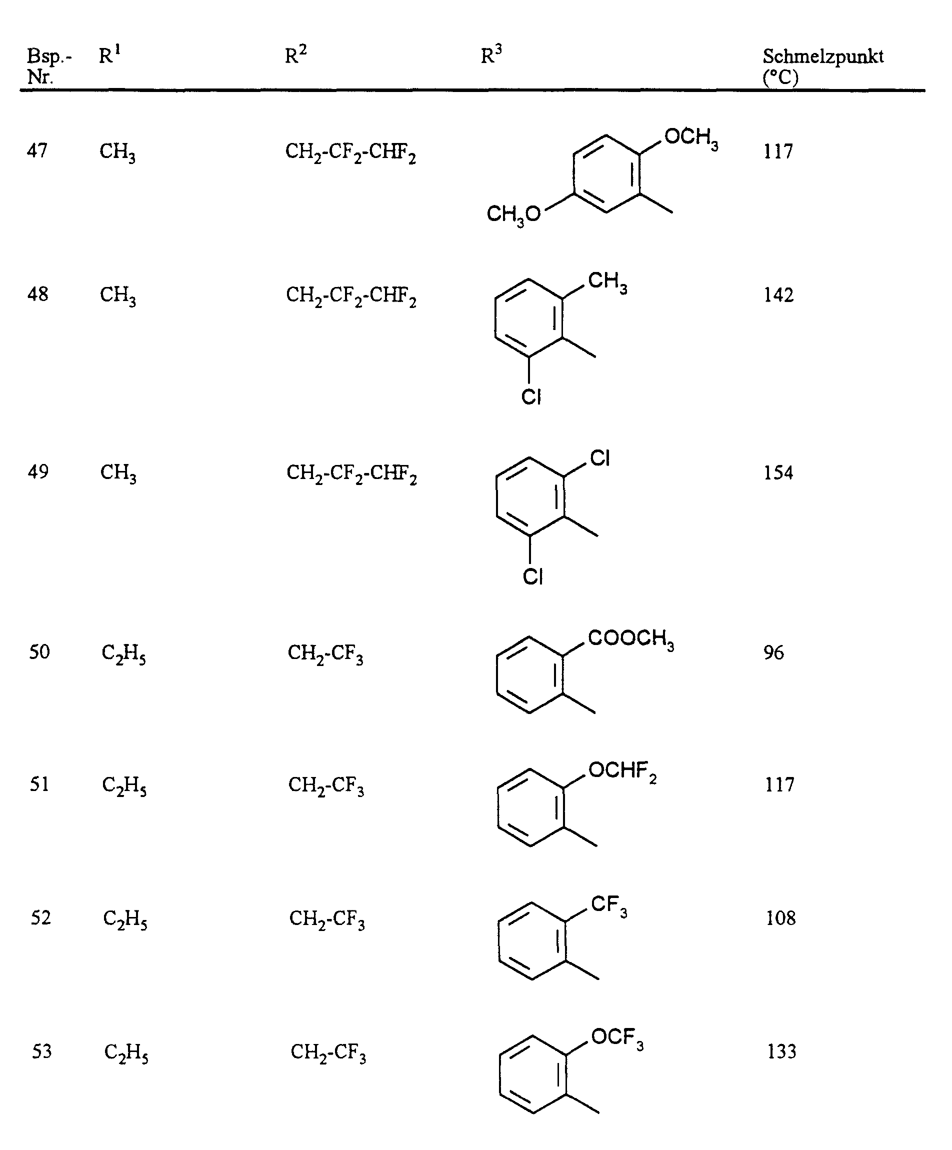

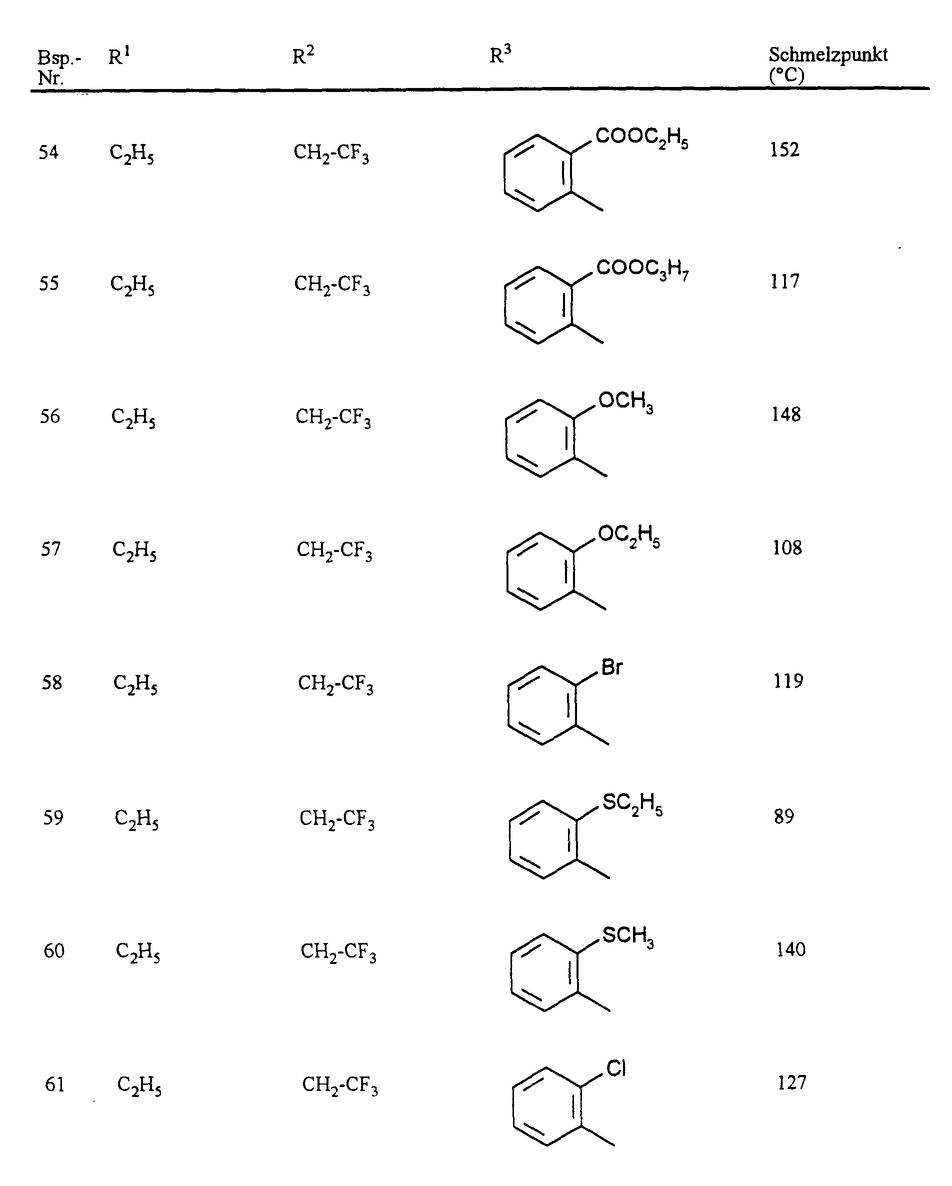

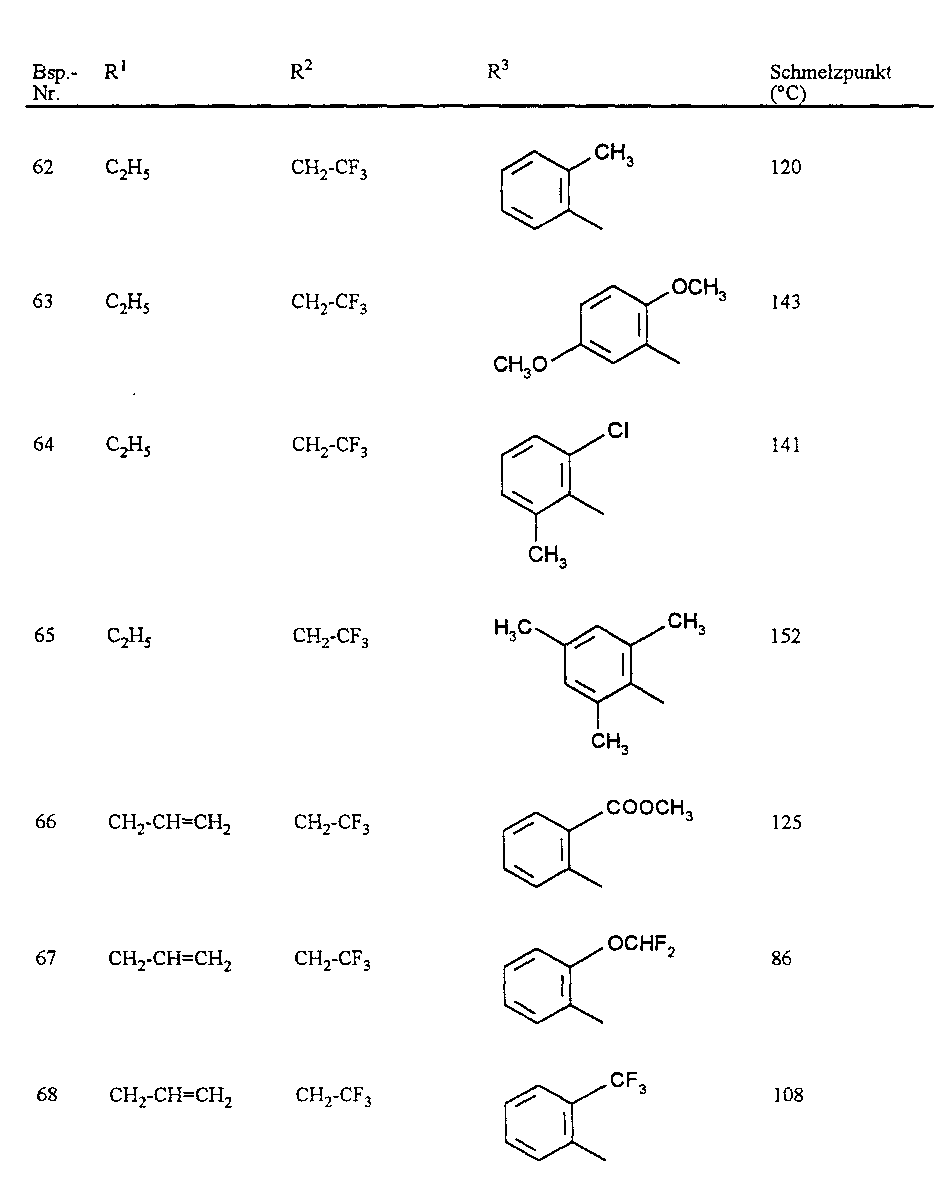

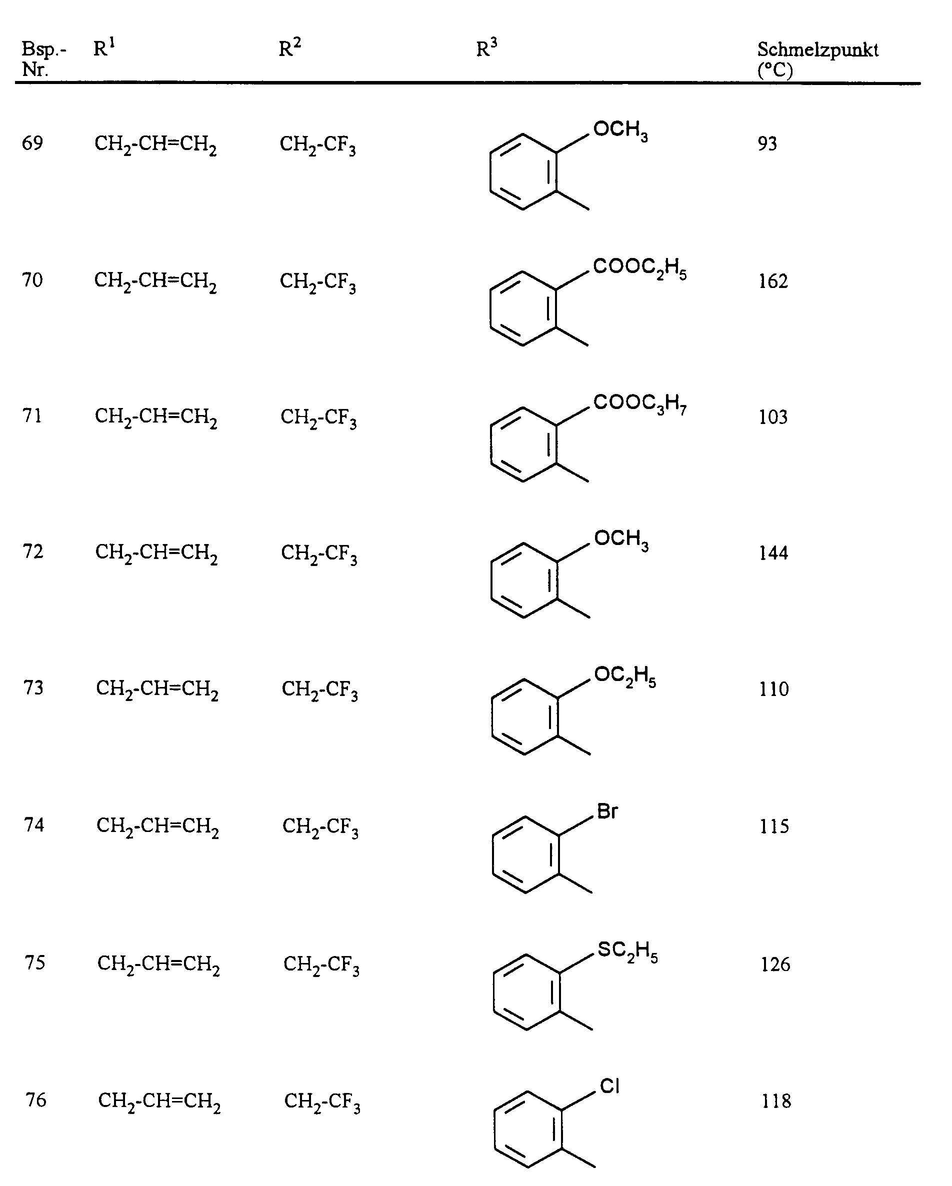

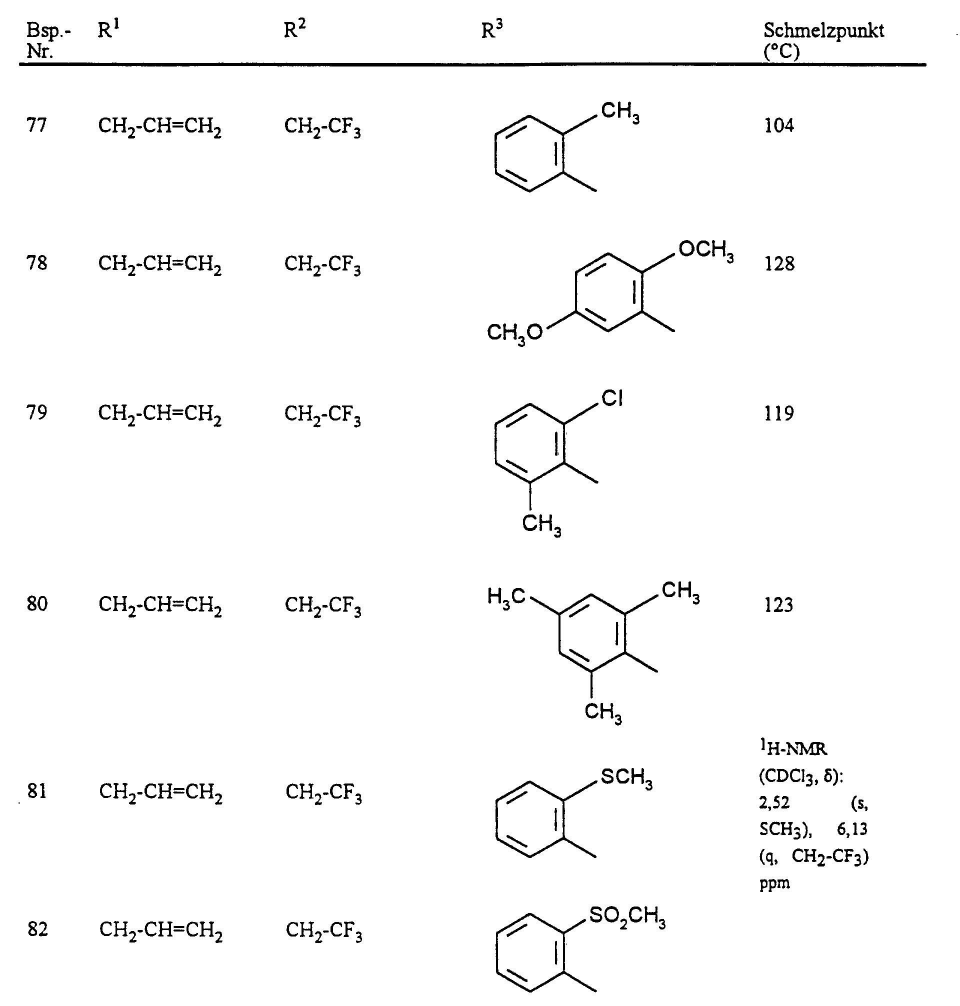

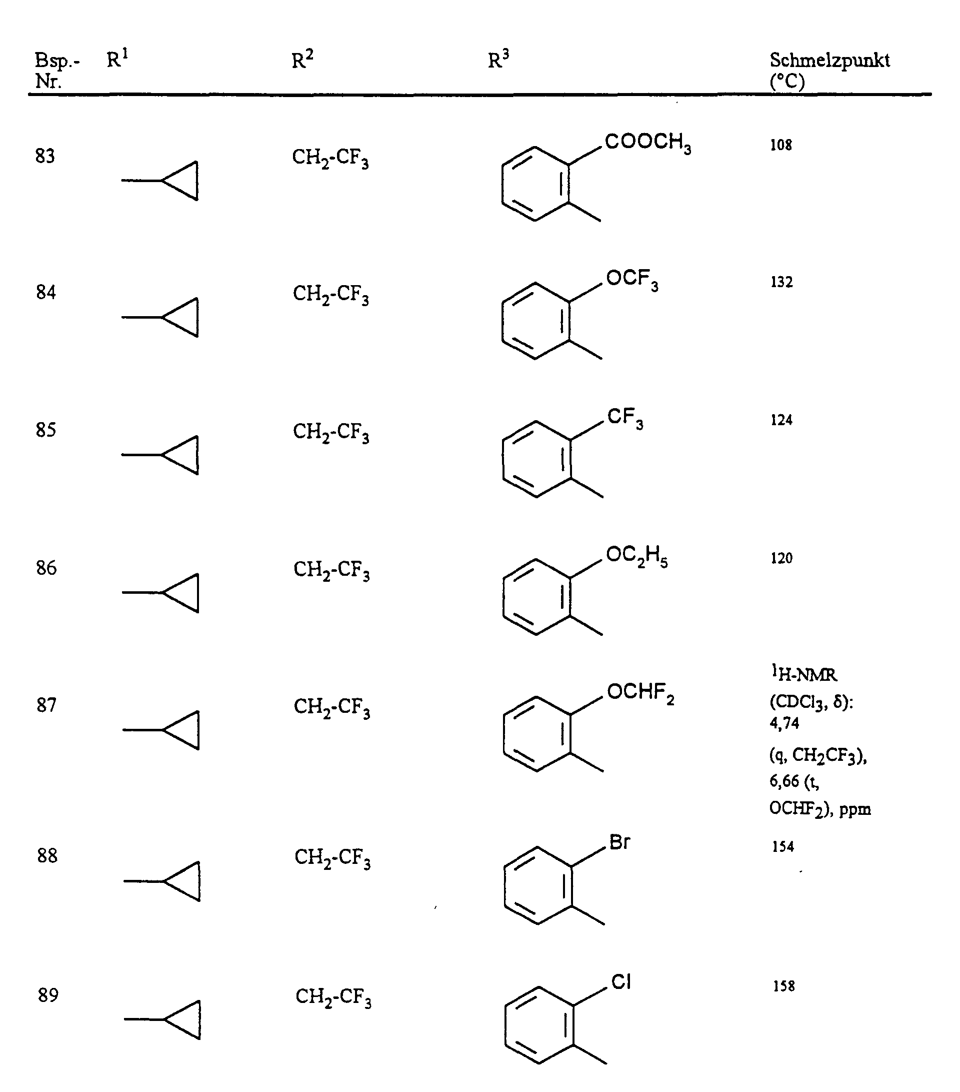

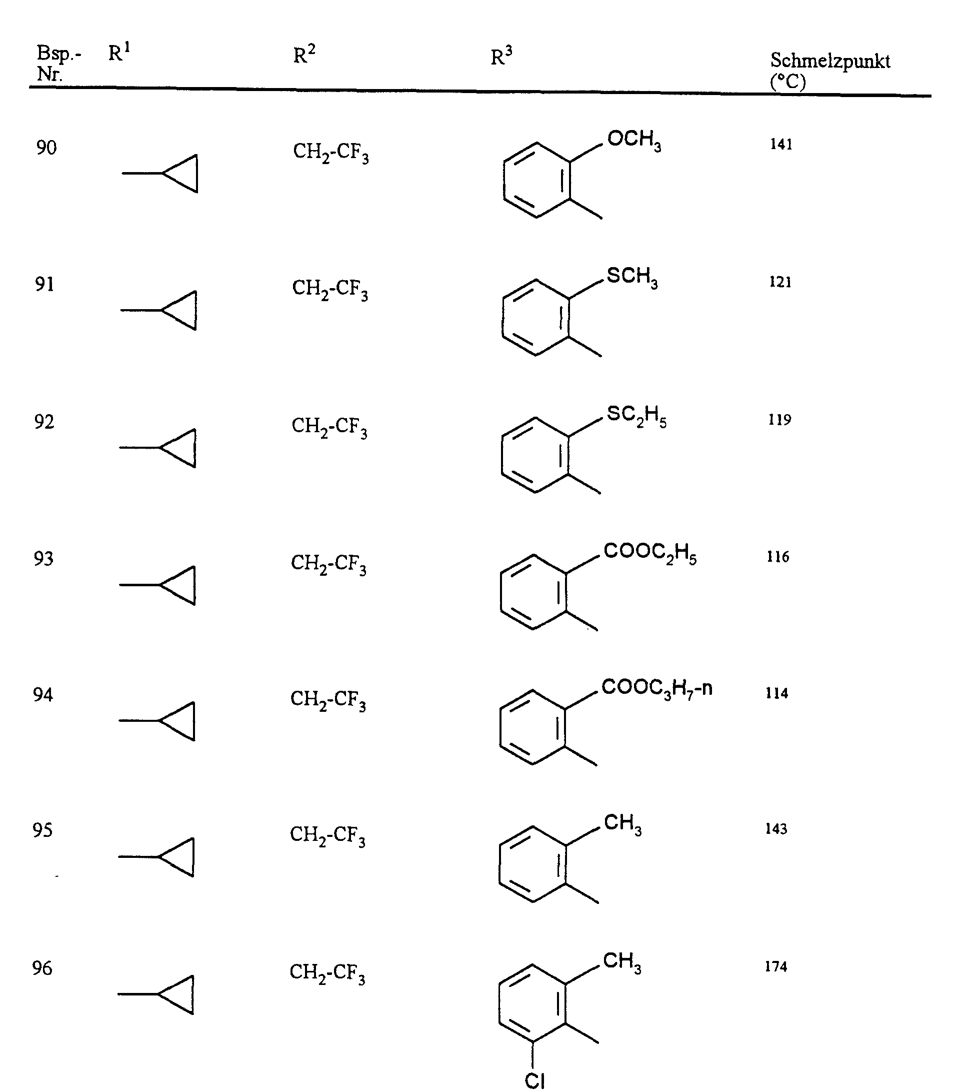

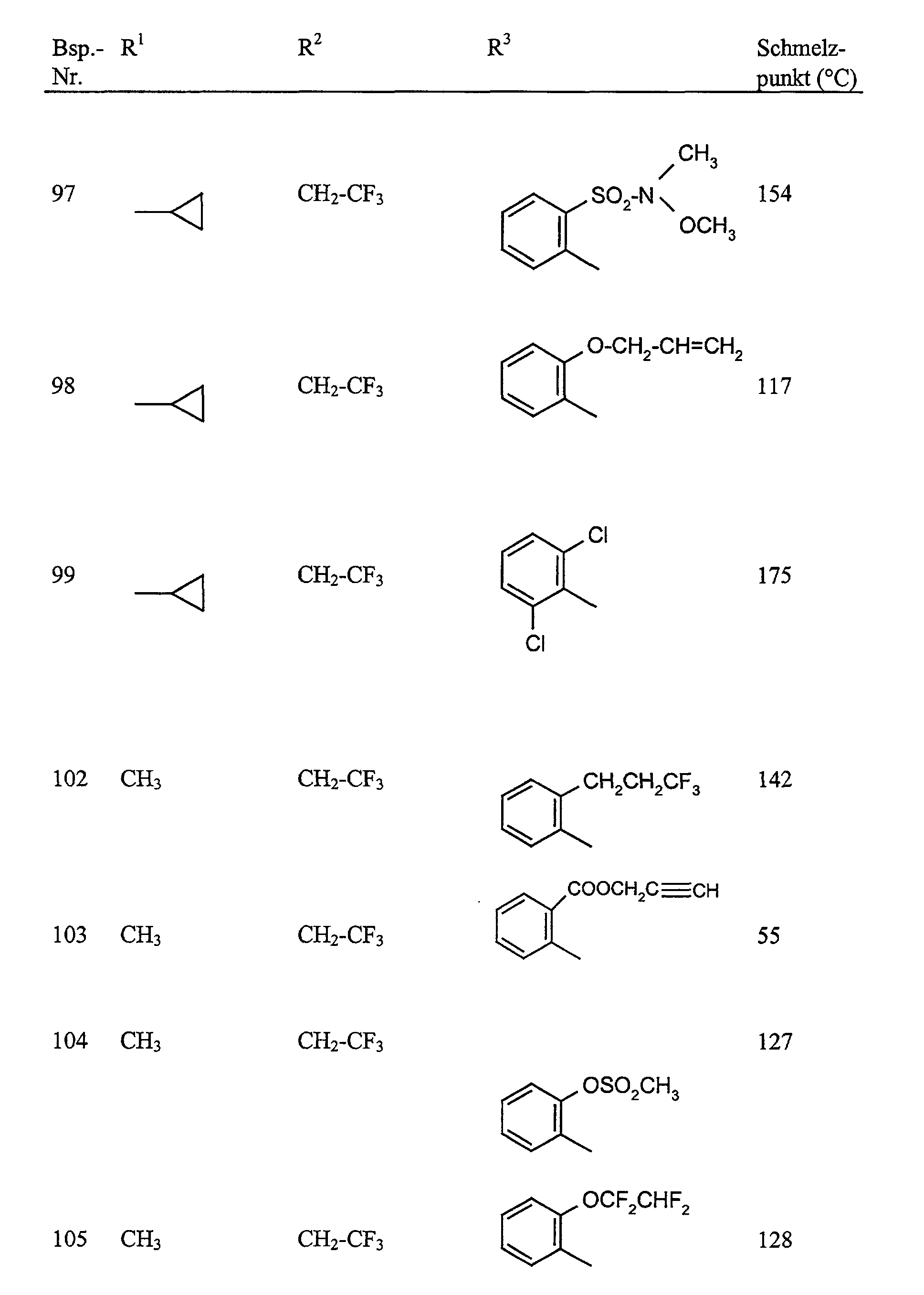

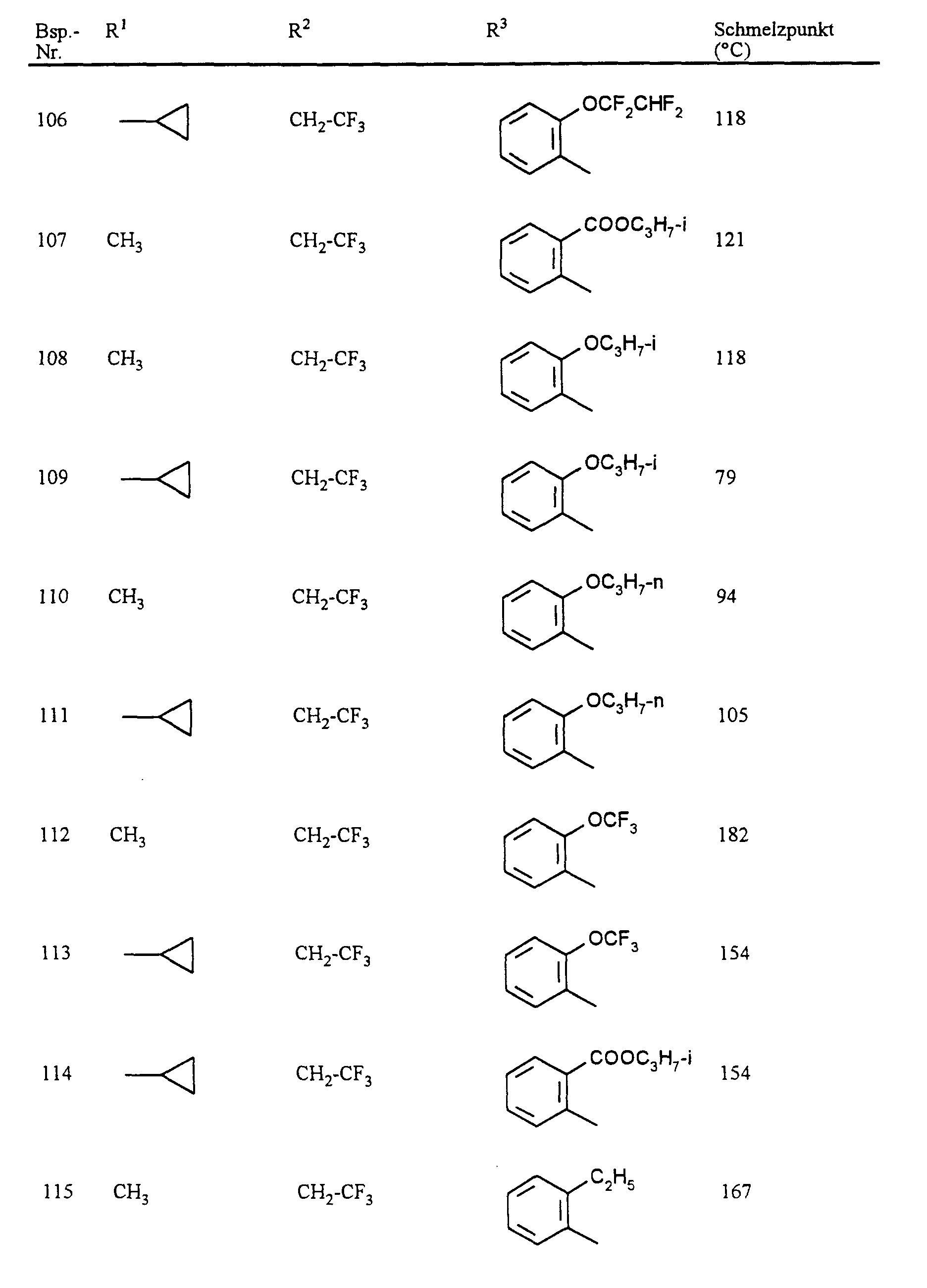

### Ausgangsstoffe der Formel (II):

### Beispiel (II-1)

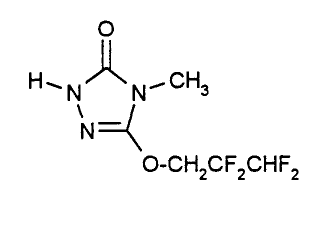

### Stufe 1: Dibutylzinn-bis-2,2,3,3-tetrafluorpropylat

723 g (2,45 Mol) Dibutylzinn-dimethylat werden mit 777 g (5,89 Mol) 2,2,3,3-Tetrafluor-propanol unter Stickstoff auf 120°C erhitzt. Dabei wird das freiwerdende Methanol weitgehend über eine Kolonne abdestilliert. Dann werden das restliche Methanol und überschüssiges 2,2,3,3-Tetrafluor-propanol im Wasserstrahlvakuum (ca. 15 mbar) weitgehend entfernt, wobei das Dibutylzinn-bis-2,2,3,3-tetrafluorpropylat als Rückstand verbleibt.

### Stufe 2: Methyliminokohlensäure-bis-(2,2,3,3-tetrafluorpropyl)-ester

Zum wie oben beschrieben erhaltenen Dibutylzinn-bis-2,2,3,3-tetrafluorpropylat werden 175 g (2,39 Mol) Methylisothiocyanat bei 40°C tropfenweise gegeben und die Mischung wird dann unter Stickstoff 36 Stunden auf 120°C erhitzt. Anschließend wird erst im Wasserstrahlvakuum, dann im Ölpumpenvakuum (Endtemperatur im Sumpf: 170°C bei 1 mbar) grob destilliert. Durch Rektifikation im Ölpumpenvakuum werden 555 g (77% der Theorie) Methyliminokohlensäure-bis-(2,2,3,3-tetrafluorpropyl)-ester erhalten.
Siedepunkt: 73°C (15 mbar), Gehalt: 98,3%.

### Stufe 3: 4-Methyl-5-(2,2,3,3-tetrafluor-propoxy)-2,4-dihydro-3H-1,2,4-triazol-3-on

555 g (1,83 Mol) Methyliminokohlensäure-bis-(2,2,3,3-tetrafluorpropyl)-ester werden in 500 ml Tetrahydrofuran gelöst und unter Eiskühlung (0°C bis 5°C) mit 18,7 g (183 mMol) Pivalinsäure (Katalysator) und 278 g (1,83 Mol) Carbazinsäure-phenylester versetzt. Das Gemisch wird 2 Stunden bei 20°C gerührt und dann auf 50°C erwärmt, wobei ein kaum noch rührbarer Kristallbrei entsteht. Bei Erhitzen auf 80°C entsteht dann wieder eine homogene Lösung. Bei ca. 200 mbar werden zunächst weitgehend das Tetrahydrofuran und dann bei ca. 15 mbar 2,2,3,3-Tetrafluor-propanol und Phenol weitgehend abdestilliert. Der Rückstand wird dann bei einer Kopftemperatur von 130°C und einem Druck von ca. 0,5 mbar weitgehend von restlichen flüchtigen Komponenten befreit und anschließend in 400 ml heißem Toluol aufgenommen. Nach Abkühlen auf 0°C wird das kristallin angefallene Produkt durch Absaugen isoliert.

Man erhält 280 g (67% der Theorie) 4-Methyl-5-(2,2,3,3-tetrafluor-propoxy)-2,4-dihydro-3H-1,2,4-triazol-3-on vom Schmelzpunkt 101°C.

### Beispiel (II-2), (nicht erfindungsgemäß)

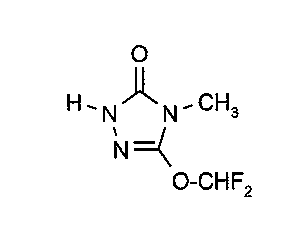

11,85 g (0,10 Mol) 4-Methylurazol werden in einer Mischung aus 100 ml Isopropanol, 25 ml Wasser und 12 g 45%iger Natronlauge (0,30 Mol NaOH) gelöst. Bei 40°C wird langsam unter Rühren Difluorchlormethan (Frigen 22) eingeleitet. Nach Einleiten von 27,7 g (0,41 Mol) wird die Reaktion unterbrochen und der Reaktionsansatz abgekühlt. Die Isopropanolphase wird abgetrennt und im Vakuum eingeengt. Der Rückstand wird mit Diethylether verrührt und von unlöslichen Bestandteilen abfiltriert. Nach Einengen der Etherphase im Vakuum wird der verbliebene Rückstand durch präparative Schichtchromatographie (Fließmittel Methylenchlorid/Methanol = 20/3) gereinigt.

Man erhält 0,9 g (5,5% der Theorie) 5-Difluormethoxy-4-methyl-2,4-dihydro-3H-1,2,4-triazol-3-on vom Schmelzpunkt 111°C.

Analog zu den Beispielen (II-1) und (II-2) können beispielsweise auch die in der nachstehenden Tabelle 2 aufgeführten Verbindungen der Formel (II) hergestellt werden.

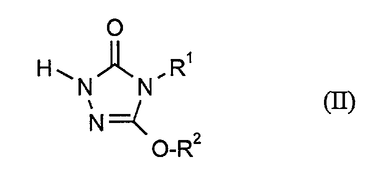

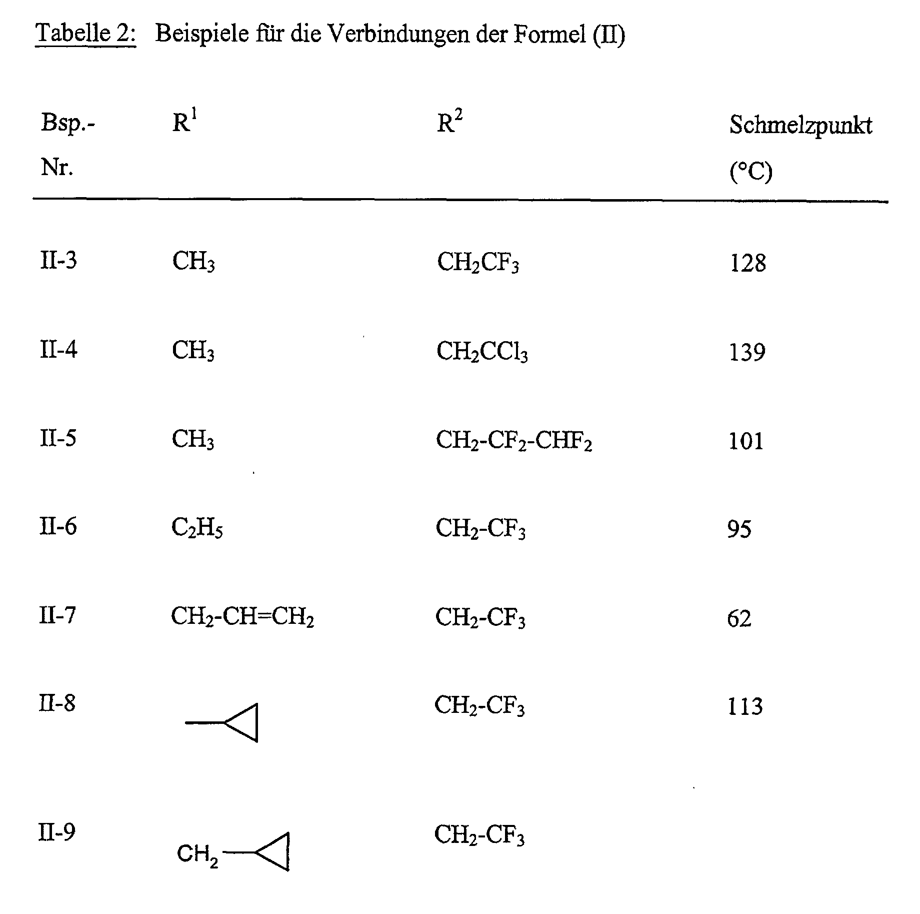

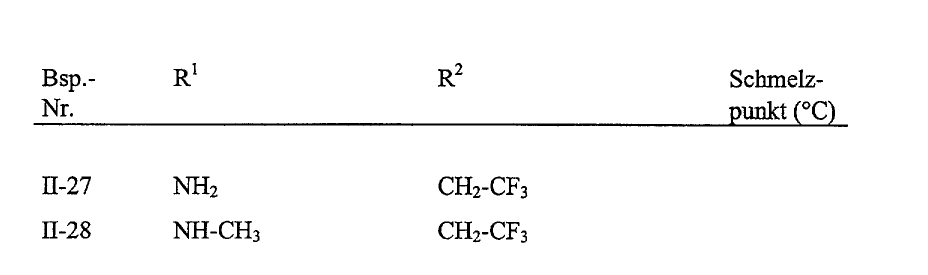

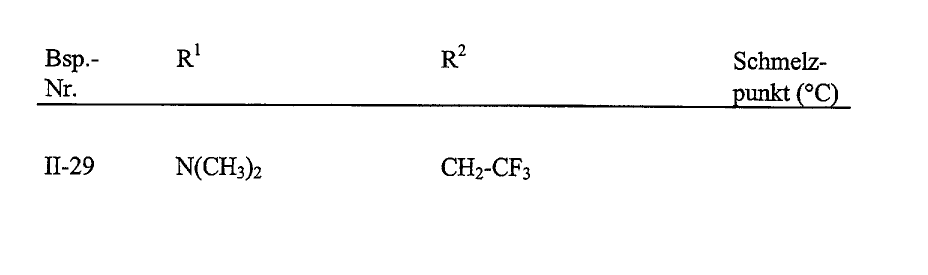

### Ausgangsstoffe der Formel (IV):

### Beispiel (IV-1)

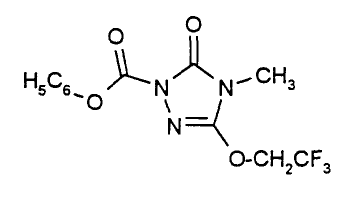

Zu einer Mischung aus 59,1 g (0,30 Mol) 4-Methyl-5-(2,2,2-trifluor-ethoxy)-2,4-dihydro-3H-1,2,4-triazol-3-on, 100 ml Wasser, 400 ml Methylenchlorid, 13,2 g (0,33 Mol) Natriumhydroxid und 1,0 g Tetrabutylammonium-bromid werden unter gutem Rühren bei 20°C 51,7 g (0,33 Mol) Chlorameisensäure-phenylester zugetropft. Nach beendeter Zugabe wird noch 16 Stunden weitergerührt, dann die organische Phase abgetrennt, mit Wasser gewaschen, getrocknet und eingeengt. Der Rückstand wird mit Diisopropylether zur Kristallisation gebracht.

Man erhält 87,6 g (92% der Theorie) 4-Methyl-2-phenoxycarbonyl-5-(2,2,2-trifluor-ethoxy)-2,4-dihydro-3H-1,2,4-triazol-3-on vom Schmelzpunkt 105°C.

Analog Beispiel (IV-1) können beispielsweise auch die in der nachstehenden Tabelle 3 aufgeführten Verbindungen der Formel (IV) hergestellt werden.

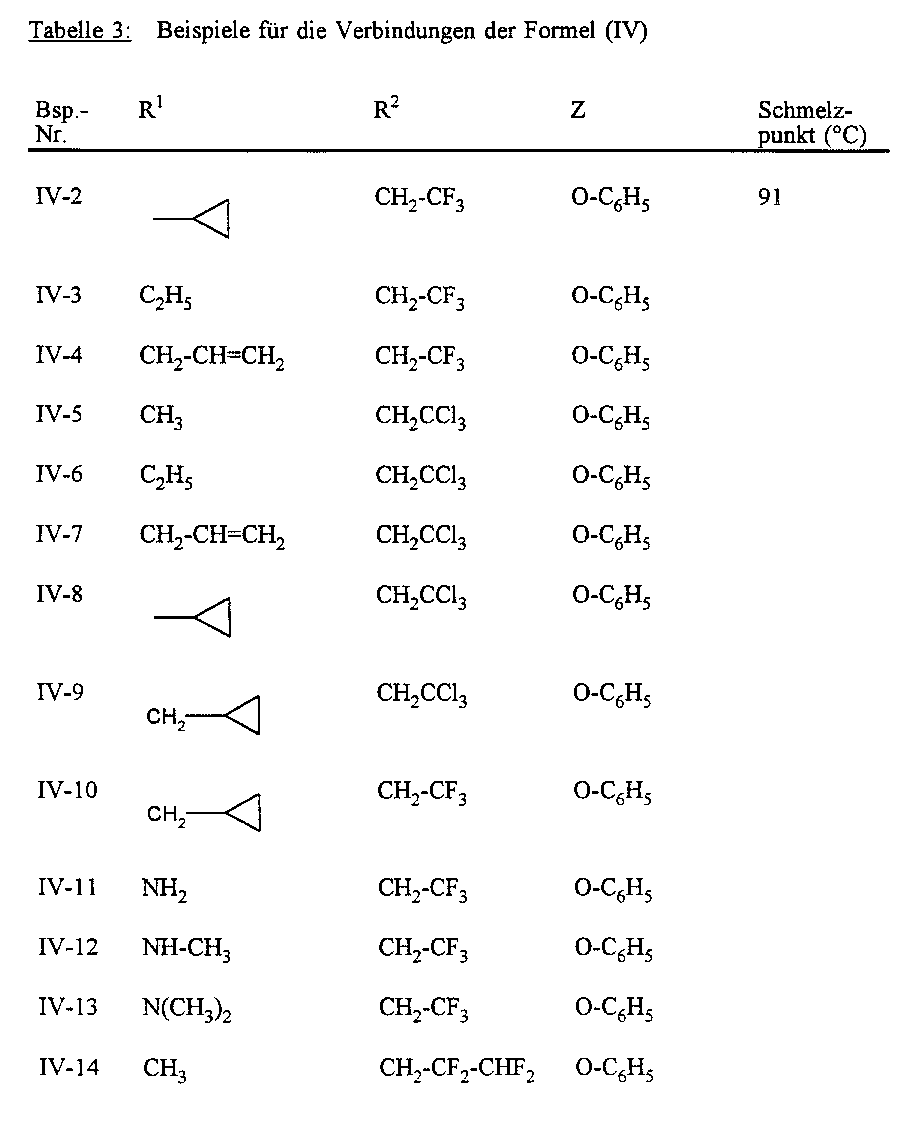

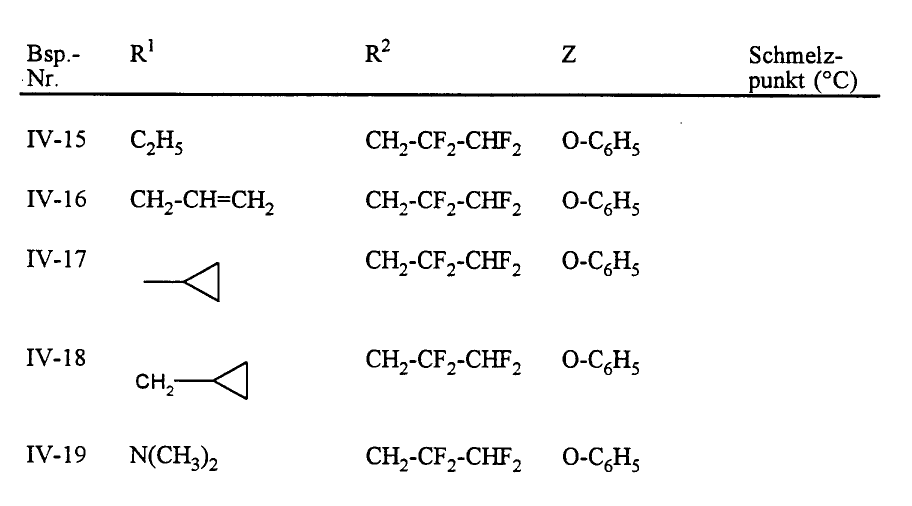

### Anwendungsbeispiele:

### Beispiel A

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
- 0 % =: keine Wirkung (wie unbehandelte Kontrolle)
- 100 % =: totale Vernichtung

In diesem Test zeigen beispielsweise die Verbindungen gemäß den Herstellungsbeispielen 1, 2, 3, 4, 5, 6, 9, 11, 12, 15, 16, 17, 25, 50, 54, 66, 83, 84, 85, 86, 87, 88, 89, 91, 93, 94, 95, 96, 97, 98 und 99 starke Wirkung gegen Unkräuter.

**Tabelle A:**

| Pre-emergence-Test/Gewächshaus | | | | | | | | | |
|---|---|---|---|---|---|---|---|---|---|
| Verbindung gemäß Herstellungs-beispiel Nr. | Aufwandmenge (g/ha) | Bromus | Lolium | Poa | Amaranthus | Galinsoga | Matricaria | Sinapis | Solanum |
| 83 | 125 | 95 | 95 | 95 | 90 | 95 | 90 | 90 | 95 |
| 84 | 125 | 90 | 90 | 90 | 90 | 95 | 90 | 90 | 95 |
| 85 | 125 | 90 | 95 | 90 | 90 | 95 | 95 | 90 | 95 |
| 86 | 125 | 95 | 80 | 90 | 90 | 95 | 95 | 95 | 95 |
| 1 | 125 | 95 | 95 | 95 | 90 | 90 | 90 | 80 | 95 |
| 3 | 125 | 95 | 90 | 80 | 80 | 70 | 95 | 80 | 90 |
| 4 | 125 | 95 | 95 | 95 | 95 | 95 | 90 | 95 | 95 |
| 5 | 125 | 95 | - | 80 | 95 | 95 | 95 | 95 | 95 |
| 6 | 125 | 95 | 50 | 95 | 95 | 70 | 60 | 90 | 95 |
| 50 | 125 | 95 | 90 | 90 | 90 | 80 | 90 | 90 | 90 |
| 54 | 125 | 95 | 90 | 90 | 90 | 95 | 90 | 70 | 90 |
| 87 | 125 | 90 | 90 | 90 | 90 | 70 | 50 | 80 | 90 |
| 88 | 125 | 90 | 90 | 90 | 80 | 95 | 95 | 80 | 90 |
| 89 | 125 | 90 | 80 | 80 | 90 | 95 | 95 | 80 | 90 |
| 91 | 125 | 90 | 70 | 80 | 80 | 80 | 70 | 80 | 90 |
| 9 | 125 | 95 | 95 | 95 | 95 | 95 | 100 | 80 | 95 |
| 11 | 125 | - | 60 | 50 | 90 | 100 | 100 | 80 | 70 |
| 12 | 125 | 80 | 80 | 80 | 95 | 100 | 80 | 80 | 80 |
| 15 | 125 | 95 | - | 60 | 95 | 70 | 95 | 90 | 90 |
| 16 | 125 | 80 | 90 | 95 | 95 | 60 | 80 | 90 | 90 |
| 93 | 125 | 95 | 95 | 95 | 95 | 95 | 95 | 95 | 95 |
| 94 | 125 | 90 | 90 | 90 | 80 | 95 | 90 | 80 | 90 |
| 95 | 125 | 80 | 90 | - | 90 | 95 | 90 | 90 | 90 |
| 96 | 125 | 95 | 95 | 90 | 90 | 90 | 90 | 90 | 80 |
| 97 | 250 | 95 | 95 | 90 | 95 | 95 | 95 | 90 | 95 |
| 2 | 250 | 95 | 95 | 95 | 95 | 95 | 80 | 80 | 95 |
| 17 | 125 | 95 | 70 | 80 | 90 | 90 | 90 | 80 | 90 |
| 98 | 125 | 90 | 70 | 80 | 90 | 90 | 80 | 80 | 90 |
| 25 | 125 | - | - | 90 | 60 | 100 | 95 | 90 | 70 |
| 66 | 125 | 90 | 95 | 90 | 70 | 95 | 95 | 70 | 95 |
| 99 | 125 | 90 | 90 | 95 | 100 | 70 | 50 | 90 | 90 |

### Beispiel B

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
- 0 % =: keine Wirkung (wie unbehandelte Kontrolle)
- 100 % =: totale Vernichtung

In diesem Test zeigen beispielsweise die Verbindungen gemäß den Herstellungsbeispielen 1, 2, 4, 5, 9, 16, 17, 25, 50, 66, 83, 84, 85, 86, 87, 88, 89, 93, 94, 95, 96, 98 und 99 starke Wirkung gegen Unkräuter.

**Tabelle B:**

| Post-emergence-test/Gewächshaus | | | | | | | | | | |
|---|---|---|---|---|---|---|---|---|---|---|
| Verbindung gemäß Herstellungs-beispiel Nr. | Aufwandmenge (g/ha) | Alopecurus | Avena | Lolium | Amaranthus | Datura | Galium | Helianthus | Solanum | Veronica |
| 83 | 250 | 90 | 90 | 95 | 90 | 90 | 90 | 90 | 90 | 90 |
| 84 | 250 | - | 95 | 70 | 70 | 90 | 80 | 90 | 90 | 95 |
| 85 | 250 | - | - | 95 | 70 | 90 | 90 | 90 | 80 | 95 |
| 86 | 500 | - | 80 | 90 | 100 | 90 | 90 | 100 | 95 | 100 |
| 1 | 250 | 90 | 90 | 100 | 80 | 80 | 70 | 90 | 90 | 95 |
| 4 | 250 | 90 | 70 | 90 | 80 | 90 | 70 | 90 | 90 | 90 |
| 5 | 250 | 80 | 50 | - | 95 | 90 | 70 | 95 | 90 | 100 |
| 87 | 125 | 70 | 80 | 50 | 80 | 95 | 70 | 80 | 90 | 90 |
| 50 | 60 | 80 | 50 | 80 | 95 | 50 | 85 | - | 90 | 70 |
| 88 | 250 | 80 | - | 70 | 95 | 95 | 90 | 100 | 90 | 90 |
| 89 | 125 | 50 | - | 80 | 95 | 95 | 90 | 95 | 95 | 95 |
| 9 | 60 | 100 | 95 | 80 | 95 | 90 | 90 | 80 | 95 | 90 |
| 16 | 125 | - | 50 | 70 | 95 | 80 | 50 | 100 | 95 | 70 |
| 93 | 125 | 95 | 90 | 80 | 95 | 95 | 90 | 100 | 95 | 70 |
| 94 | 125 | 80 | 90 | 80 | 70 | 90 | 70 | 80 | 90 | - |
| 95 | 60 | 70 | 70 | - | 90 | - | 95 | 70 | 70 | 80 |
| 17 | 125 | 50 | - | - | 90 | 80 | 50 | 100 | 95 | 90 |
| 98 | 125 | - | - | - | 95 | 50 | - | 100 | 80 | 90 |
| 96 | 60 | 95 | 70 | 90 | 95 | 70 | 90 | 80 | - | 50 |
| 2 | 125 | 60 | 60 | 50 | 80 | 80 | - | 95 | 80 | 80 |
| 25 | 60 | 60 | 50 | - | 80 | 80 | 50 | 100 | 90 | - |
| 66 | 60 | 50 | - | - | 80 | 80 | 50 | 100 | 90 | - |
| 99 | 60 | 60 | 50 | 50 | 90 | 95 | 95 | 60 | 90 | 70 |

### Beispiel C

### Podosphaera-Test (Apfel) / protektiv

| | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen durch Bestäuben mit Konidien des Apfelmehltauerregers Podosphaera leucotricha inokuliert.

Die Pflanzen werden dann im Gewächshaus bei 23°C und einer relativen Luftfeuchtigkeit von ca. 70 % aufgestellt.

10 Tage nach der Inokulation erfolgt die Auswertung.

In diesem Test zeigen z.B. die Verbindungen gemäß Herstellungsbeispielen 1, 3, 83 und 85 bei einer Wirkstoffkonzentration von 25ppm einen Wirkungsgrad von 87 bis 100 %.

**Tabelle C:**

| Podosphaera-Test (Apfel) / protektiv | |
|---|---|
| Wirkstoff (Verbindung gemäß Herstellungs-beispiel Nr.) | Wirkungsgrad in % der unbehandelten Kontrolle bei einer Wirkstoffkonzentration von 25 ppm |
| 1 | 89 |
| 3 | 87 |
| 83 | 100 |
| 85 | 99 |

### Beispiel D

### Sphaerotheca-Test (Gurke) / protektiv

| | |
|---|---|
| Lösungsmittel | 4,7 GewichtsteileAceton |
| Emulgator | 0,3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

In diesem Test zeigt z.B. die Verbindung gemäß Herstellungsbeispiel 50 bei einer Wirkstoffkonzentration von 100 ppm einen Wirkungsgrad von 92 %.

**Tabelle D**

| Sphaerotheca-Test (Gurke) / protektiv | |
|---|---|
| Wirkstoff (Verbindung gemäß Herstellungs-beispiel Nr.) | Wirkungsgrad in % der unbehandelten Kontrolle bei einer Wirkstoffkonzentration von 100 ppm |
| 50 | 92 |

## Patentansprüche

1. Sulfonylaminocarbonyltriazolinone mit Halogenalkoxy-Substituenten der allgemeinen Formel (I), 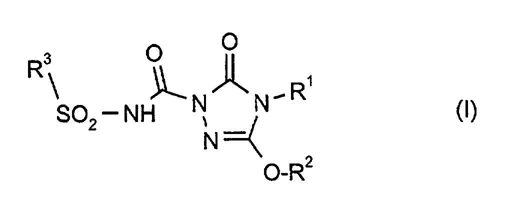 in welcher
R¹ für Wasserstoff, Hydroxy, Amino, C₁-C₆-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor und/oder Chlor substituiertes C₁-C₆-Alkoxy oder C₂-C₆-Alkenyloxy, für jeweils gegebenenfalls durch Fluor und/oder Chlor substituiertes C₁-C₆-Alkylamino, Di-(C₁-C₄-alkyl)-amino oder C₁-C₄-Alkanoylamino, für jeweils gegebenenfalls durch Fluor, Chlor, Brom und/oder C₁-C₄-Alkyl substituiertes C₃-C₆-Cycloalkyl oder C₃-C₆-Cycloalkyl-C₁-C₄-alkyl, oder für jeweils gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl oder Phenyl-C₁-C₄-alkyl steht,
R² für -CH₂-CF₃, -CH₂-CCl₃ oder -CH₂-CF₂-CHF₂ steht und
R³ für die Gruppierung 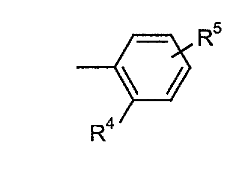 steht,
worin
R⁴ und R⁵ gleich oder verschieden sind und für Wasserstoff, Fluor, Chlor, Brom, Iod, Nitro, C₁-C₆-Alkyl (welches gegebenenfalls durch Fluor, Chlor, Brom, Cyano, Carboxy, C₁-C₄-Alkoxycarbonyl, C₁-C₄-Alkylamino-carbonyl, Di-(C₁-C₄-alkyl)amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆-Cycloalkyl oder Phenyl substituiert ist), für C₂-C₆-Alkenyl (welches gegebenenfalls durch Fluor, Chlor, Brom, Cyano, C₁-C₄-Alkoxy-carbonyl, Carboxy oder Phenyl substituiert ist), für C₂-C₆-Alkinyl (welches gegebenenfalls durch Fluor, Chlor, Brom, Cyano, C₁-C₄-Alkoxy-carbonyl, Carboxy oder Phenyl substituiert ist), für C₁-C₄-Alkoxy (welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist), für C₁-C₄-Alkylthio (welches gegebenenfalls durch Fluor, Chlor, Brom, Cyano, Carboxy, C₁-C₄-Alkoxycarbonyl, C₁-C₄-Alkylthio, C₁-C₄-Alkylsulfinyl oder C₁-C₄-Alkylsulfonyl substituiert ist), für C₂-C₆-Alkenyloxy (welches gegebenenfalls durch Fluor, Chlor, Brom, Cyano oder C₁-C₄-Alkoxy-carbonyl substituiert ist), für C₂-C₆-Alkenylthio (welches gegebenenfalls durch Fluor, Chlor, Brom, Cyano, Nitro, C₁-C₃-Alkylthio oder C₁-C₄-Alkoxycarbonyl substituiert ist), C₃-C₆-Alkinyloxy, C₃-C₆-Alkinylthio oder für den Rest - S(O)ₚ-R⁶ stehen, wobei
p für die Zahlen 1 oder 2 steht und
R⁶ für C₁-C₄-Alkyl (welches gegebenenfalls durch Fluor, Chlor, Brom, Cyano oder C₁-C₄-Alkoxy-carbonyl substituiert ist), C₃-C₆-Alkenyl, C₃-C₆-Alkinyl, C₁-C₄-Alkoxy, C₁-C₄-Alkoxy-C₁-C₄-alkylamino, C₁-C₄-Alkylamino, Di-(C₁-C₄-alkyl)-amino, Phenyl oder für den Rest -NHOR⁷ steht, wobei
R⁷ für C₁-C₁₂-Alkyl (welches gegebenenfalls durch Fluor, Chlor, Cyano, C₁-C₄-Alkoxy, C₁-C₄-Alkylthio, C₁-C₄-Alkylsulfinyl, C₁-C₄-Alkylsulfonyl, C₁-C₄-Alkylcarbonyl, C₁-C₄-Alkoxy- carbonyl, C₁-C₄-Alkylaminocarbonyl oder Di-(C₁-C₄-alkyl)-amino-carbonyl substituiert ist), für C₃-C₆-Alkenyl (welches gegebenenfalls durch Fluor, Chlor oder Brom substituiert ist), C₃-C₆-Alkinyl, C₃-C₆-Cycloalkyl, C₃-C₆-cycloalkyl-C₁-C₂alkyl, Phenyl-C₁-C₂-alkyl (welches gegebenenfalls durch Fluor, Chlor, Nitro, Cyano, C₁-C₄-Alkyl, C₁-C₄-Alkoxy oder C₁-C₄-Alkoxy-carbonyl substituiert ist), für Benzhydryl oder für Phenyl (welches gegebenenfalls durch Fluor, Chlor, Nitro, Cyano, C₁-C₄-Alkyl, Trifluormethyl, C₁-C₄-Alkoxy, C₁-C₂-Fluoralkoxy, C₁-C₄-Alkylthio, Trifluormethylthio oder C₁-C₄-Alkoxycarbonyl substituiert ist) steht,
R⁴ und/oder R⁵ weiterhin für Phenyl oder Phenoxy, für C₁-C₄-Alkylcarbonylamino, C₁-C₄-Alkoxy-carbonylamino, C₁-C₄-Akloxyaminocarbonyl-amino, Di-(C₁-C₄-alkyl)-amino-carbonylamino, oder für den Rest CO-R⁸ stehen, wobei
R⁸ für Wasserstoff, C₁-C₆-Alkyl, C₃-C₆-Cycloalkyl, C₁-C₆-Alkoxy, C₃-C₆-Cycloalkoxy, C₃-C₆-Alkenyloxy, C₃-C₆-Alkinyloxy, C₁-C₄-Alkylthio, C₁-C₄-Alkylamino, C₁-C₄-Alkoxyamino, C₁-C₄-Alkoxy-C₁-C₄-alkylamino oder Di-(C₁-C₄-alkyl)-amino steht (welche gegebenenfalls durch Fluor und/oder Chlor substituiert sind),
R⁴ und/oder R⁵ weiterhin für Trimethylsilyl, Thiazolinyl, C₁-C₄-Alkylsulfonyloxy, Di-(C₁-C₄-alkyl)-aminosulfonylamino oder für den Rest
-CH=N-R⁹ stehen, wobei
R⁹ für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/oder Chlor substituiertes C₁-C₆-Alkoxy, C₃-C₆-Alkenoxy, C₃-C₆-Alkinoxy oder Benzyloxy für Amino, C₁-C₄-Alkylamino, Di-(C₁-C₄-alkyl)-amino, Phenylamino, C₁-C₄-Alkyl-carbonyl-amino, C₁-C₄-Alkoxy-carbonylamino, C₁-C₄-Alkyl-sulfonylamino oder für gegebenenfalls durch Fluor, Chlor, Brom oder Methyl substituiertes Phenylsulfonylamino steht,
weiterhin
R³ für den Rest 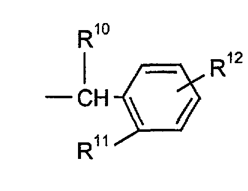 steht,
worin
R¹⁰ für Wasserstoff oder C₁-C₄-Alkyl steht,
R¹¹ und R¹² gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), Carboxy, C₁-C₄-Alkoxycarbonyl, Dimethylaminocarbonyl, C₁-C₄-Alkylsulfonyl oder Di-(C₁-C₄-alkyl)-aminosulfonyl stehen;
weiterhin
R³ für den Rest 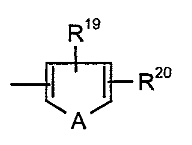 steht,
worin
R¹⁹ und R²⁰ gleich oder verschieden sind und für Wasserstoff, Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), C₁-C₄-Alkylthio, C₁-C₄-Alkylsulfinyl oder C₁-C₄-Alkylsulfonyl (welches gegebenenfalls durch Fluor und/oder Chlor substituiert ist), Di-(C₁-C₄-alkyl)-amino-sulfonyl, C₁-C₄-Alkoxy-carbonyl oder Dimethylaminocarbonyl stehen, und
A für Schwefel steht,
sowie deren Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium, Tri-(C₁-C₄-alkyl)-sulfonium-, C₅- oder C₆-Cycloalkyl-ammonium- und Di-(C₁-C₂-alkyl)-benzyl-ammonium-Salze.

2. Verbindungen der Formel (I) gemäß Anspruch 1, **dadurch gekennzeichnet, daß** darin
R¹ für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für jeweils gegebenenfalls durch Fluor und/oder Chlor substituiertes Methoxy, Ethoxy, n- oder i-Propoxy, n-, i-, s- oder t-Butoxy, Propenyloxy oder Butenyloxy, für Methylamino, Ethylamino, n- oder i-Propylamino, n-, i-, s- oder t-Butylamino, Dimethylamino oder Diethylamino, für jeweils gegebenenfalls durch Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Fluor, Chlor, Brom, Cyano, Methyl, Trifluormethyl oder Methoxy substituiertes Benzyl oder Phenyl steht,
R² für -CH₂-CF₃, -CH₂-CCl₃ oder -CH₂-CF₂-CHF₂ steht und
R³ für den Rest 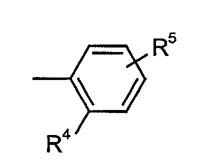 steht,
worin
R⁴ für Fluor, Chlor, Brom, Methyl, Ethyl, n- oder i-Propyl, Trifluormethyl, Methoxy, Ethoxy, n- oder i-Propoxy, Difluormethoxy, Trifluormethoxy, 2-Chlor-ethoxy, 2-Methoxy-ethoxy, Methylthio, Ethylthio, n- oder i-Propylthio, Methylsulfinyl, Ethylsulfinyl, Methylsulfonyl, Ethylsulfonyl, Dimethylaminosulfonyl, Diethylaminosulfonyl, N-Methoxy-N-methylaminosulfonyl, Phenyl, Phenoxy, Methoxycarbonyl, Ethoxycarbonyl, n- oder i-Propoxycarbonyl, steht und
R⁵ für Wasserstoff, Methyl, Ethyl, Fluor, Chlor oder Brom steht;
weiterhin
R³ für den Rest 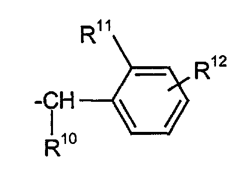 steht,
worin
R¹⁰ für Wasserstoff steht,
R¹¹ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
R¹² für Wasserstoff steht;
weiterhin
R³ für den Rest 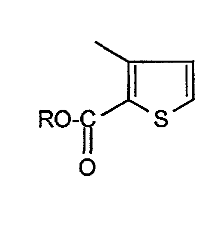 steht,
worin
R für Methyl, Ethyl, n- oder i-Propyl steht.

3. Verfahren zur Herstellung von Verbindungen der Formel (I) gemäß Anspruch 1 und deren Salzen, **dadurch gekennzeichnet, daß** man
(a) Triazolinone der allgemeinen Formel (II) 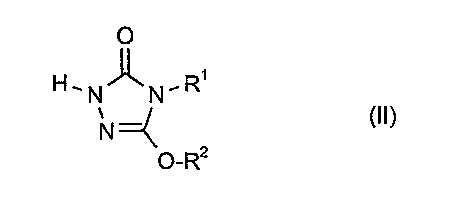 in welcher
R¹ und R² die in Anspruch 1 angegebene Bedeutung haben,
mit Sulfonylisocyanaten der allgemeinen Formel (III)
R³-SO₂-N=C=O (III)
in welcher
R³ die in Anspruch 1 angegebene Bedeutung hat, gegebenenfalls in Gegenwart eines Reaktionshilfsmittels und gegebenenfalls in Gegenwart eines Verdünnungsmittels umsetzt,
oder daß man
(b) Triazolinon-Derivate der allgemeinen Formel (IV) 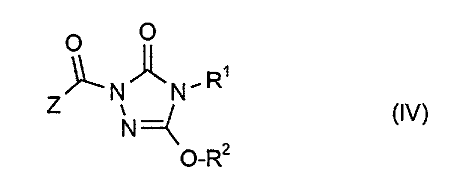 in welcher
R¹ und R² die oben angegebene Bedeutung haben und
Z für Halogen, Alkoxy, Aralkoxy oder Aryloxy steht,
mit Sulfonsäureamiden der allgemeinen Formel (V)
R³-SO₂-NH₂ (V)
in welcher
R³ die oben angegebene Bedeutung hat,
gegebenenfalls in Gegenwart eines Säureakzeptors und gegebenenfalls in Gegenwart eines Verdünnungsmittels umsetzt,
oder daß man
(c) Triazolinone der allgemeinen Formel (II) 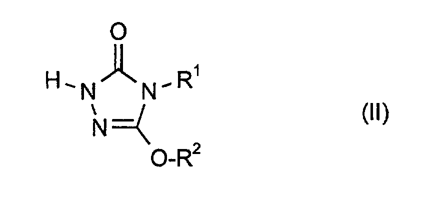 in welcher
R¹ und R² die oben angegebene Bedeutung haben,
mit Sulfonsäureamid-Derivaten der allgemeinen Formel (VI)
R³-SO₂-NH-CO-Z (VI)
in welcher
R³ die oben angegebene Bedeutung hat und
Z für Halogen, Alkoxy, Aralkoxy oder Aryloxy steht,
gegebenenfalls in Gegenwart eines Säureakzeptors und gegebenenfalls in Gegenwart eines Verdünnungsmittels umsetzt,
oder daß man
(d) Triazolinone der allgemeinen Formel (II) 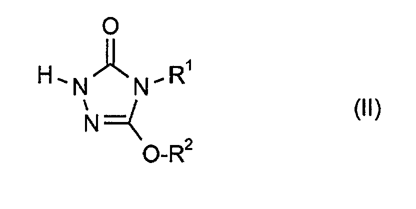
in welcher
R¹ und R² die oben angegebene Bedeutung haben,
mit Sulfonsäurehalogeniden der allgemeinen Formel (VII)
R³-SO₂-X (VII)
in welcher
R³ die oben angegebene Bedeutung hat und
X für Halogen steht,
und Metallcyanaten der allgemeinen Formel (VIII)
MOCN (VIII)
in welcher
M für ein Alkalimetall oder Erdalkalimetall-äquivalent steht,
gegebenenfalls in Gegenwart eines Reaktionshilfsmittels und gegebenenfalls in Gegenwart eines Verdünnungsmittels umsetzt,
und gegebenenfalls die nach Verfahren (a), (b), (c) oder (d) erhaltenen Verbindungen der Formel (I) nach üblichen Methoden in Salze überführt.

4. Herbizide und fungizide Mittel, **gekennzeichnet durch** einen Gehalt an mindestens einer Verbindung der Formel (I) gemäß Anspruch 1.

5. Verwendung von Verbindungen der allgemeinen Formel (I) gemäß Anspruch 1 zur Bekämpfung von unerwünschtem Pflanzenwachstum und/oder von phytopathogenen Pilzen.

6. Verfahren zur Bekämpfung von Unkräutern und/oder phytopathogenen Pilzen, **dadurch gekennzeichnet, daß** man Verbindungen der allgemeinen Formel (I) gemäß Anspruch 1 auf die Unkräuter bzw. Pilze oder ihren Lebensraum einwirken läßt.

7. Verfahren zur Herstellung von herbiziden oder fungiziden Mitteln, **dadurch gekennzeichnet, daß** man Verbindungen der allgemeinen Formel (I) gemäß Anspruch 1 mit Streckmitteln und/oder oberflächenaktiven Mitteln vermischt.

8. Triazolinone der allgemeinen Formel (II) 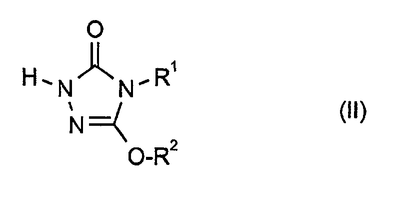 in welcher
R¹ und R² für jeweils die in Anspruch 1 angegebenen Bedeutungen haben.

9. Triazolinon-Derivate der allgemeinen Formel (IV) 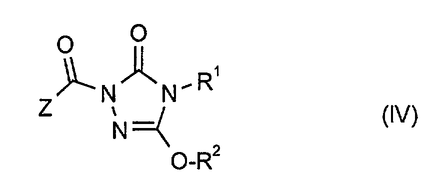 in welcher
R¹ und R² jeweils die in Anspruch 1 angegebenen Bedetungen haben, und
Z für Fluor, Chlor, Brom, Methoxy, Ethoxy, Benzyloxy, Phenoxy, Halogen- oder Nitrophenoxy steht.

## Claims

1. Sulphonylaminocarbonyltriazolinones having halogenoalkoxy substituents, of the general formula (I), 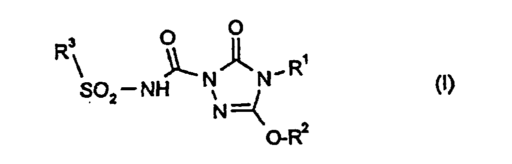 in which
R¹ represents hydrogen, hydroxyl, amino, C₁-C₆-alkylidenamino, represents optionally fluorine-, chlorine-, bromine-, cyano-, C₁-C₄-alkoxy-, C₁-C₄-alkyl-carbonyl-substituted C₁-C₆-alkyl, represents in each case optionally fluorine- or chlorine- and/or bromine-substituted C₂-C₆-alkenyl or C₂-C₆-alkinyl, represents in each case optionally fluorine- and/or chlorine-substituted C₁-C₆-alkoxy or C₂-C₆-alkenyloxy, represents in each case optionally fluorine- and/or chlorine-substituted C₁-C₆-alkylamino, di-(C₁C₄-alkyl)-amino or C₁-C₄-alkanoylamino, represents in each case optionally fluorine-, chlorine-, bromine- and/or C₁-C₄-alkyl-substituted C₃-C₆-cycloalkyl or C₃-C₆-cycloalkyl-C₁-C₄-alkyl, or represents in each case optionally fluorine-, chlorine-, bromine-, cyano-, nitro-, C₁-C₄-alkyl-, trifluoromethyl-, C₁-C₄-alkoxy- and/or C₁-C₄-alkoxy-carbonyl-substituted phenyl or phenyl-C₁-C₄-alkyl,
R² represents -CH₂-CF₃, -CH₂-CCl₃ or -CH₂-CF₂-CHF₂ and
R³ represents the grouping 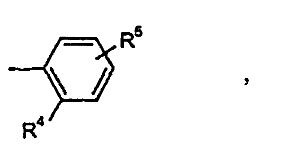 in which
R⁴ and R⁵ are identical or different and represent hydrogen, fluorine, chlorine, bromine, iodine, nitro, C₁-C₆-alkyl (which is optionally substituted by fluorine, chlorine, bromine, cyano, carboxyl, C₁C₄-alkoxycarbonyl, C₁-C₄-alkylamino-carbonyl, di-(C₁-C₄-alkyl)amino-carbonyl, hydroxyl, C₁-C₄-alkoxy, formyloxy, C₁-C₄-alkyl-carbonyloxy, C₁-C₄-alkoxy-carbonyloxy, C₁-C₄-alkylamino-carbonyloxy, C₁-C₄-alkylthio, C₁-C₄-alkylsulphinyl, C₁-C₄-alkylsulphonyl, di-(C₁-C₄-alkyl)-aminosulphonyl, C₃-C₆-cycloalkyl or phenyl), represent C₂-C₆-alkenyl (which is optionally substituted by fluorine, chlorine, bromine, cyano, C₁-C₄-alkoxy-carbonyl, carboxyl or phenyl), represents C₂C₆-alkinyl (which is optionally substituted by fluorine, chlorine, bromine, cyano, C₁-C₄-alkoxy-carbonyl, carboxyl or phenyl), represent C₁-C₄-alkoxy (which is optionally substituted by fluorine, chlorine, bromine, cyano, carboxyl, C₁-C₄-alkoxy-carbonyl, C₁-C₄-alkoxy, C₁-C₄-alkylthio, C₁-C₄-alkylsulphinyl or C₁-C₄-alkylsulphonyl), represent C₁-C₄-alkylthio (which is optionally substituted by fluorine, chlorine, bromine, cyano, carboxyl, C₁-C₄-alkoxy-carbonyl, C₁-C₄-alkylthio, C₁-C₄-alkylsulphinyl or C₁-C₄-alkylsulphinyl), represent C₂-C₆-alkenyloxy (which is optionally substituted by fluorine, chlorine, bromine, cyano or C₁-C₄-alkoxy-carbonyl), represent C₂-C₆-alkenylthio (which is optionally substituted by fluorine, chlorine, bromine, cyano, nitro, C₁-C₃-alkylthio or C₁-C₄-alkoxycarbonyl), C₃-C₆-alkinyloxy, C₃-C₆-alkinylthio or the radical -S(O)ₚ-R⁶, where
p represents the numbers 1 or 2 and
R⁶ represents C₁-C₄-alkyl (which is optionally substituted by fluorine, chlorine, bromine, cyano or C₁-C₄-alkoxy-carbonyl), C₃-C₆-alkenyl, C₃-C₆-alkinyl, C₁-C₄-alkoxy, C₁-C₄-alkoxy-C₁-C₄-alkylamino, C₁-C₄-alkylamino, di-(C₁-C₄-alkyl)-amino, phenyl or represents the radical -NHOR⁷, where
R⁷ represents C₁-C₁₂-alkyl (which is optionally substituted by fluorine, chlorine, cyano, C₁-C₄-alkoxy, C₁-C₄-alkylthio, C₁-C₄-alkylsulphinyl, C₁-C₄-alkylsulphonyl, C₁-C₄-alkyl-carbonyl, C₁-C₄-alkoxy-carbonyl, C₁-C₄-alkylamino-carbonyl or di-(C₁-C₄-alkyl)-amino-carbonyl), represents C₃-C₆-alkenyl (which is optionally substituted by fluorine, chlorine or bromine), C₃-C₆-alkinyl, C₃-C₆-cycloalkyl, C₃-C₆-cycloalkyl-C₁-C₂-alkyl, phenyl-C₁-C₂-alkyl (which is optionally substituted by fluorine, chlorine, nitro, cyano, C₁-C₄-alkyl, C₁-C₄-alkoxy or C₁-C₄-alkoxy-carbonyl), represents benzhydryl or represents phenyl (which is optionally substituted by fluorine, chlorine, nitro, cyano, C₁-C₄-alkyl, trifluoromethyl, C₁-C₄-alkoxy, C₁-C₂-fluoroalkoxy, C₁-C₄-alkylthio, trifluoromethyl or C₁-C₄-alkoxy-carbonyl),
R⁴ and/or R⁵ furthermore represent phenyl or phenoxy, represent C₁-C₄-alkyl-carbonylamino, C₁-C₄-alkoxy-carbonylamino, C₁-C₄-alkoxyamino-carbonyl-amino, di-(C₁-C₄-alkyl)-amino-carbonylamino, or represent the radical CO-R⁸, where
R⁸ represents hydrogen, C₁-C₆-alkyl, C₃-C₆-cycloalkyl, C₁-C₆-alkoxy, C₃-C₆-cycloalkoxy, C₃-C₆-alkenyloxy, C₃-C₆-alkinyloxy, C₁-C₄-alkylthio, C₁-C₄-alkylamino, C₁-C₄-alkoxyamino, C₁-C₄-alkoxy-C₁-C₄-alkylamino or di-(C₁-C₄-alkyl)-amino (which are optionally substituted by fluorine and/or chlorine),
R⁴ and/or R⁵ furthermore represent trimethylsilyl, thiazolinyl, C₁-C₄-alkyl-sulphonyloxy, di-(C₁-C₄-alkyl)-aminosulfonylamino or represent the radical
-CH=N-R⁹,
where
R⁹ represents optionally fluorine-, chlorine-, cyano-, carboxyl-, C₁-C₄-alkoxy-, C₁-C₄-alkylthio, C₁-C₄-alkylsulphinyl- or C₁-C₄-alkylsulphonyl-substituted C₁-C₆-alkyl, represents optionally fluorine- or chlorine-substituted benzyl, represents optionally fluorine- or chlorine-substituted C₃-C₆-alkenyl or C₃-C₆-alkinyl, represents optionally fluorine-, chlorine-, bromine-, C₁-C₄-alkyl-, C₁-C₄-alkoxy-, trifluoromethyl-, trifluoromethoxy- or trifluoromethylthio-substituted phenyl, represents optionally fluorine- and/or chlorine-substituted C₁-C₆-alkoxy, C₃-C₆-alkenoxy, C₃-C₆-alkinoxy or benzyloxy, represents amino, C₁-C₄-alkylamino, di-(C₁-C₄-alkyl)-amino, phenylamino, C₁-C₄-alkyl-carbonyl-amino, C₁-C₄-alkoxy-carbonylamino, C₁-C₄-alkyl-sulphonylamino or represents optionally fluorine-, chlorine-, bromine- or methyl-substituted phenylsulphonylamino,
furthermore
R³ represents the radical 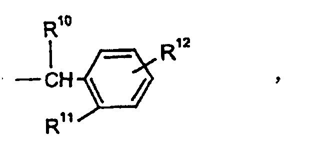 in which
R¹⁰ represents hydrogen or C₁-C₄-alkyl,
R¹¹ and R¹² are identical or different and represent hydrogen, fluorine, chlorine, bromine, nitro, cyano, C₁-C₄-alkyl (which is optionally substituted by fluorine and/or chlorine), C₁-C₄-alkoxy (which is optionally substituted by fluorine and/or chlorine), carboxyl, C₁-C₄-alkoxy-carbonyl, dimethylaminocarbonyl, C₁-C₄-alkylsulphonyl or di-(C₁-C₄-alkyl)-aminosulphonyl;
furthermore
R³ presents the radical 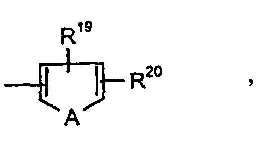 in which
R¹⁹ and R²⁰ are identical or different and represent hydrogen, fluorine, chlorine, bromine, cyano, nitro, C₁-C₄-alkyl (which is optionally substituted by fluorine and/or chlorine), C₁-C₄-alkoxy (which is optionally substituted by fluorine and/or chlorine), C₁-C₄-alkylthio, C₁-C₄-alkylsulphinyl or C₁-C₄-alkylsulphonyl (which is optionally substituted by fluorine and/or chlorine), di-(C₁-C₄-alkyl)-amino-sulphonyl, C₁-C₄-alkoxy-carbonyl or dimethylaminocarbonyl, and
A represents sulphur,
and their sodium, potassium, magnesium, calcium, ammonium, C₁-C₄-alkyl-ammonium, di-(C₁-C₄-alkyl)-ammonium, tri-(C₁-C₄-alkyl)-ammonium, tetra-(C₁-C₄-alkyl)-ammonium, tri-(C₁-C₄-alkyl)-sulphonium, C₅- or C₆-cycloalkyl-ammonium and di-(C₁-C₂-alkyl)-benzyl-ammonium salts.

2. Compounds of the formula (I) according to Claim 1, **characterized in that**
R¹ represents in each case optionally fluorine-, chlorine-, cyano-, methoxy- or ethoxy-substituted methyl, ethyl, n- or i-propyl, n-, i-, s- or t-butyl, represents in each case optionally fluorine-, chlorine- or bromine-substituted propenyl, butenyl, propinyl and butinyl, represents in each case optionally fluorine- and/or chlorine-substituted methoxy, ethoxy, n- or i-propoxy, n-, i-, s- or t-butoxy, propenyloxy or butenyloxy, represents methylamino, ethylamino, n- or i-propylamino, n-, i-, s- or t-butylamino, dimethylamino or diethylamino, represents in each case optionally fluorine-, chlorine-, bromine-, methyl- or ethyl-substituted cyclopropyl, cyclobutyl, cyclopentyl, cyclohexyl, cyclopropylmethyl, cyclobutylmethyl, cyclopentylmethyl or cyclohexylmethyl, or represents in each case optionally fluorine-, chlorine-, bromine-, cyano-, methyl-, trifluoromethyl- or methoxy-substituted benzyl or phenyl,
R² represents -CH₂-CF₃, -CH₂-CCl₃ or -CH₂-CF₂-CHF₂ and
R³ represents the radical 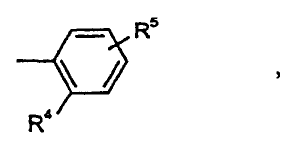 in which
R⁴ represents fluorine, chlorine, bromine, methyl, ethyl, n- or i-propyl, trifluoromethyl, methoxy, ethoxy, n- or i-propoxy, difluoromethoxy, trifluoromethoxy, 2-chloro-ethoxy, 2-methoxy-ethoxy, methylthio, ethylthio, n- or i-propylthio, methylsulphinyl, ethylsulphinyl, methylsulphonyl, ethylsulphonyl, dimethylaminosulphonyl, diethylaminosulphonyl, N-methoxy-N-methylaminosulphonyl, phenyl, phenoxy, methoxycarbonyl, ethoxycarbonyl, n- or i-propoxycarbonyl, and
R⁵ represents hydrogen, methyl, ethyl, fluorine, chlorine or bromine;
furthermore
R³ represents the radical 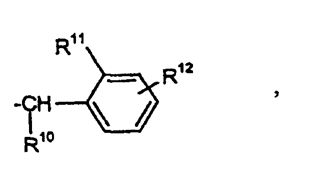 in which
R¹⁰ represents hydrogen,
R¹¹ represents fluorine, chlorine, bromine, methyl, methoxy, difluoromethoxy, trifluoromethoxy, ethoxy, methoxycarbonyl, ethoxycarbonyl, methylsulphonyl or dimethylaminosulphonyl and
R¹² represents hydrogen;
furthermore
R³ represents the radical 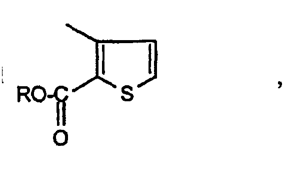 in which
R represents methyl, ethyl, n- or i-propyl.

3. Process for preparing compounds of the formula (I) according to Claim 1 and salts thereof, **characterized in that**
(a) triazolinones of the general formula (II) 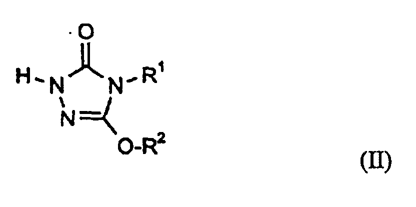 in which
R¹ and R² have the meaning given in Claim 1,
are reacted with sulphonyl isocyanates of the general formula (III)
R³-SO₂-N=C=O (III)
in which
R³ has the meaning given in Claim 1,
if appropriate in the presence of a reaction auxiliary and if appropriate in the presence of a diluent,
or
(b) triazolinone derivatives of the general formula (IV) 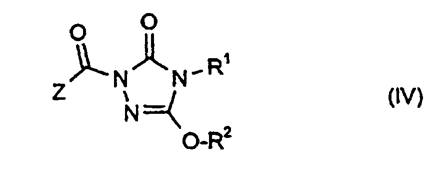 in which
R¹ and R² have the meaning given above and
Z represents halogen, alkoxy, aralkoxy or aryloxy,
are reacted with sulphonamides of the general formula (V)
R³-SO₂-NH₂ (V)
in which
R³ has the meaning given above,
if appropriate in the presence of an acid acceptor and if appropriate in the presence of a diluent,
or
(c) triazolinones of the general formula (II) 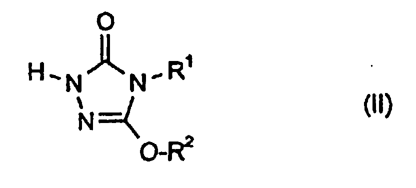 in which
R¹ and R² have the meaning given above,
are reacted with sulphonamide derivatives of the general formula (VI)
R³-SO₂-NH-CO-Z (VI)
in which
R³ is the meaning given above and
Z represents halogen, alkoxy, aralkoxy or aryloxy,
if appropriate in the presence of an acid acceptor and if appropriate in the presence of a diluent,
or
(d) triazolinones of the general formula (II) 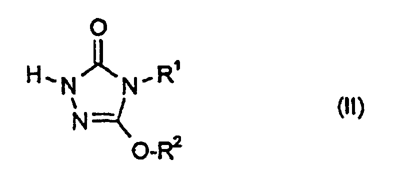 in which
R¹ and R² have the meaning given above,
are reacted with sulphonyl halides of the general formula (VII)
R³-SO₂-X (VII)
in which
R³ has the meaning given above and
X represents halogen,
and metal cyanates of the general formula (VIII)
MOCN (VIII)
in which
M represents an alkali metal or an alkaline earth metal equivalent,
if appropriate in the presence of a reaction auxiliary and if appropriate in the presence of a diluent,
and the compounds of the formula (I) obtained according to processes (a), (b), (c) or (d) are, if appropriate, converted by customary methods into salts.

4. Herbicidal and fungicidal compositions, **characterized in that** they comprise at least one compound of the formula (I) according to Claim 1.

5. Use of compounds of the general formula (I) according to Claim 1 for controlling undesirable vegetation and/or phytopathogenic fungi.

6. Method for controlling weeds and/or phytopathogenic fungi, **characterized in that** compounds of the general formula (I) according to Claim 1 are allowed to act on the weeds and/or fungi or their habitat.

7. Process for preparing herbicidal or fungicidal compositions, **characterized in that** compounds of the general formula (I) according to Claim 1 are mixed with extenders and/or surfactants.

8. Triazolinones of the general formula (II) 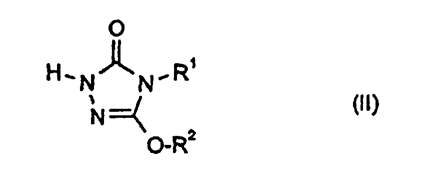 in which
R¹ and R² each have the meanings given in Claim 1.

9. Triazolinone derivatives of the general formula (IV) 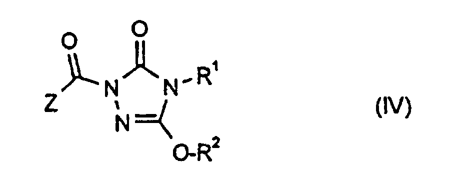 in which
R¹ and R² each have the meanings given in Claim 1 and
Z represents fluorine, chlorine, bromine, methoxy, ethoxy, benzyloxy, phenoxy, halogeno or nitrophenoxy.

## Revendications

1. Sulfonylaminocarbonyltriazolinones portant des substituants halogénalkoxy, de formule générale (I) 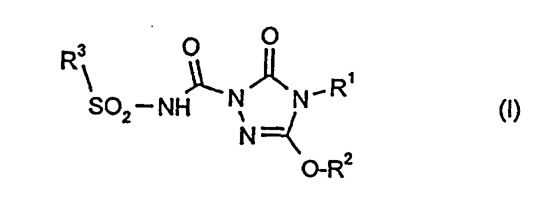 dans laquelle
R¹ représente l'hydrogène, un groupe hydroxy, amino, (alkylidène en C₁ à C₆)amino, un groupe alkyle en C₁ à C₆ éventuellement substitué par du fluor, du chlore, du brome, un radical cyano, alkoxy en C₁ à C₄, (alkyle en C₁ à C₄)carbonyle ou (alkoxy en C₁ à C₄)carbonyle, un groupe alcényle en C₂ à C₆ ou alcynyle en C₂ à C₆ dont chacun est éventuellement substitué par du fluor, du chlore et/ou du brome, un groupe alkoxy en C₁ à C₆ ou alcényloxy en C₂ à C₆ dont chacun est éventuellement substitué par du fluor et/ou du chlore, un groupe alkylamino en C₁ à C₆, di-(alkyle en C₁ à C₄)-amino ou alcanoylamino en C₁ à C₄ dont chacun est éventuellement substitué par du fluor et/ou du chlore, un groupe cycloalkyle en C₃ à C₆ ou (cycloalkyle en C₃ à C₆)-(alkyle en C₁ à C₄) dont chacun est éventuellement substitué par du fluor, du chlore, du brome et/ou un radical alkyle en C₁ à C₄, ou bien un groupe phényle ou phényl-(alkyle en C₁ à C₄) dont chacun est éventuellement substitué par du fluor, du chlore, du brome, un radical cyano, nitro, alkyle en C₁ à C₄, trifluorométhyle, alkoxy en C₁ à C₄ et/ou (alkoxy en C₁ à C₄)carbonyle,
R² représente un groupe -CH₂-CF₃, -CH₂-CCl₃ ou -CH₂-CF₂-CHF₂ et
R³ représente le groupement 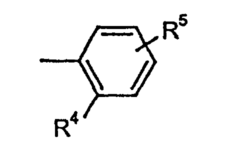 dans lequel
R⁴ et R⁵ sont identiques ou différents et représentent l'hydrogène, le fluor, le chlore, le brome, l'iode, un groupe nitro, alkyle en C₁ à C₆ (qui est éventuellement substitué par du fluor, du chlore, du brome, un radical cyano, carboxy, (alkoxy en C₁ à C₄)carbonyle, (alkylamino en C₁ à C₄)carbonyle, di-(alkyle en C₁ à C₄)aminocarbonyle, hydroxy, alkoxy en C₁ à C₄, formyloxy, (alkyle en C₁ à C₄)carbonyloxy, (alkoxy en C₁ à C₄)carbonyloxy, (alkylamino en C₁ à C₄)carbonyloxy, alkylthio en C₁ à C₄, alkylsulfinyle en C₁ à C₄, alkylsulfonyle en C₁ à C₄, di-(alkyle en C₁ à C₄)aminosulfonyle, cycloalkyle en C₃ à C₆ ou phényle), un groupe alcényle en C₂ à C₆ (qui est substitué, le cas échéant, par du fluor, du chlore, du brome, un radical cyano, (alkoxy en C₁ à C₄)carbonyle, carboxy ou phényle), un groupe alcynyle en C₂ à C₆ (qui est substitué, le cas échéant, par du fluor, du chlore, du brome, un radical cyano, (alkoxy en C₁ à C₄)carbonyle, carboxy ou phényle), un groupe alkoxy en C₁ à C₄ (qui est substitué, le cas échéant, par du fluor, du chlore, du brome, un radical cyano, carboxy, (alkoxy en C₁ à C₄)carbonyle, alkoxy en C₁ à C₄, alkylthio en C₁ à C₄, alkylsulfinyle en C₁ à C₄ ou alkylsulfonyle en C₁ à C₄), un groupe alkylthio en C₁ à C₄ (qui est substitué, le cas échéant, par du fluor, du chlore, du brome, un radical cyano, carboxy, (alkoxy en C₁ à C₄)carbonyle, alkylthio en C₁ à C₄, alkylsulfinyle en C₁ à C₄ ou alkylsulfonyle en C₁ à C₄), un groupe alcényloxy en C₂ à C₆ (qui est substitué, le cas échéant, par du fluor, du chlore, du brome, un radical cyano ou (alkoxy en C₁ à C₄)carbonyle), un groupe alcénylthio en C₂ à C₆ (qui est substitué, le cas échéant, par du fluor, du chlore, du brome, un radical cyano, nitro, alkylthio en C₁ à C₃ ou (alkoxy en C₁ à C₄)carbonyle), un groupe alcynyloxy en C₃ à C₆, alcynylthio en C₃ à C₆ ou le reste -S(O)ₚ-R⁶, dans lequel
p représente les nombres 1 ou 2 et
R⁶ est un groupe alkyle en C₁ à C₄ (qui est substitué, le cas échéant par du fluor, du chlore, du brome, un radical cyano ou (alkoxy en C₁ à C₄)carbonyle), un groupe alcényle en C₃ à C₆, alcynyle en C₃ à C₆, alkoxy en C₁ à C₄, (alkoxy en C₁ à C₄)-(alkylamino en C₁ à C₄), alkylamino en C₁ à C₄, di-(alkyle en C₁ à C₄)amino, phényle ou le reste -NHOR⁷, dans lequel
R⁷ représente un groupe alkyle en C₁ à C₁₂ (qui est substitué, le cas échéant, par du fluor, du chlore, un radical cyano, alkoxy en C₁ à C₄, alkylthio en C₁ à C₄, alkylsulfinyle en C₁ à C₄, alkylsulfonyle en C₁ à C₄, (alkyle en C₁ à C₄)-carbonyle, (alkoxy en C₁ à C₄)carbonyle, (alkylamino en C₁ à C₄)carbonyle ou di-(alkyle en C₁ à C₄)aminocarbonyle), un groupe alcényle en C₃ à C₆ (qui est substitué, le cas échéant, par du fluor, du chlore ou du brome), un groupe alcynyle en C₃ à C₆, cycloalkyle en C₃ à C₆, (cycloalkyle en C₃ à C₆)-(alkyle en C₁ ou C₂), phényl-(alkyle en C₁ ou C₂) (qui est substitué, le cas échéant, par du fluor, du chlore, un radical nitro, cyano, alkyle en C₁ à C₄, alkoxy en C₁ à C₄ ou (alkoxy en C₁ à C₄)carbonyle), un groupe benzhydryle ou un groupe phényle (qui est substitué, le cas échéant, par du fluor, du chlore, un radical nitro, cyano, alkyle en C₁ à C₄, trifluorométhyle, alkoxy en C₁ à C₄, fluoralkoxy en C₁ ou C₂, alkylthio en C₁ à C₄, trifluorométhylthio ou (alkoxy en C₁ à C₄) carbonyle),
R⁴ et/ou R⁵ représentent en outre un groupe phényle ou phénoxy, un groupe (alkyle en C₁ à C₄)carbonylamino, (alkoxy en C₁ à C₄)carbonylamino, (alkoxyamino en C₁ à C₄)carbonylamino, di-(alkyle en C₁ à C₄)aminocarbonyl-amino ou le reste CO-R⁸, dans lequel
R⁸ représente l'hydrogène, un groupe alkyle en C₁ à C₆, cycloalkyle en C₃ à C₆, alkoxy en C₁ à C₆, cycloalkoxy en C₃ à C₆, alcényloxy en C₃ à C₆, alcynyloxy en C₃ à C₆, alkylthio en C₁ à C₄, alkylamino en C₁ à C₄, alkoxyamino en C₁ à C₄, (alkoxy en C₁ à C₄)-alkylamino en C₁ à C₄ ou di-(alkyle en C₁ à C₄)amino (qui sont éventuellement substitués par du fluor et/ou du chlore),
R⁴ et/ou R⁵ représentent en outre un groupe triméthylsilyle, thiazolinyle, (alkyle en C₁ à C₄)sulfonyloxy, di-(alkyle en C₁ à C₄)aminosulfonyl-amino ou le reste
-CH=N-R⁹,
dans lequel
R⁹ représente un groupe alkyle en C₁ à C₆ éventuellement substitué par du fluor, du chlore, un radical cyano, carboxy, alkoxy en C₁ à C₄, alkylthio en C₁ à C₄, alkylsulfinyle en C₁ à C₄ ou alkylsulfonyle en C₁ à C₄, un groupe benzyle éventuellement substitué par du fluor ou du chlore, un groupe alcényle en C₃ à C₆ ou alcynyle en C₃ à C₆ éventuellement substitué par du fluor
ou du chlore, un groupe phényle éventuellement substitué par du fluor, du chlore, du brome, un radical alkyle en C₁ à C₄, alkoxy en C₁ à C₄, trifluorométhyle, trifluorométhoxy ou trifluorométhylthio, un groupe alkoxy en C₁ à C₆, alcényloxy en C₃ à C₆, alcynyloxy en C₃ à C₆ ou benzyloxy portant éventuellement un substituant fluoro et/ou chloro, un groupe amino, alkylamino en C₁ à C₄, di-(alkyle en C₁ à C₄)amino, phénylamino, (alkyle en C₁ à C₄)carbonylamino, (alkoxy en C₁ à C₄)carbonylamino, (alkyle en C₁ à C₄)sulfonylamino ou un groupe phénylsulfonylamino éventuellement substitué par du fluor, du chlore, du brome ou un radical méthyle,
en outre
R³ représente le reste 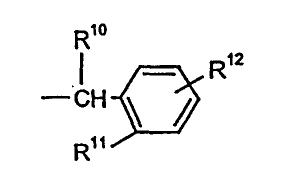 dans lequel
R¹⁰ est l'hydrogène ou un groupe alkyle en C₁ à C₄,
R¹¹ et R¹² sont identiques ou différents et représentent l'hydrogène, le fluor, le chlore, le brome, un groupe nitro, cyano, alkyle en C₁ à C₄ (qui est éventuellement substitué par du fluor et/ou du chlore), un groupe alkoxy en C₁ à C₄ (qui est éventuellement substitué par du fluor et/ou du chlore), un groupe carboxy, (alkoxy en C₁ à C₄)carbonyle, diméthylaminocarbonyle, alkylsulfonyle en C₁ à C₄ ou di-(alkyle en C₁ à C₄)aminosulfonyle ; en outre
R³ représente le reste 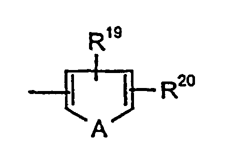 dans lequel
R¹⁹ et R²⁰ sont identiques ou différents et représentent l'hydrogène, le fluor, le chlore, le brome, un groupe cyano, nitro, alkyle en C₁ à C₄ (qui est substitué, le cas échéant, par du fluor et/ou du chlore), un groupe alkoxy en C₁ à C₄ (qui est substitué, le cas échéant, par du fluor et/ou du chlore), un groupe alkylthio en C₁ à C₄, alkylsulfinyle en C₁ à C₄ ou alkylsulfonyle en C₁ à C₄ (qui est éventuellement substitué par du fluor et/ou du chlore), un groupe di-(alkyle en C₁ à C₄)aminosulfonyle, (alkoxy en C₁ à C₄)carbonyle ou diméthylaminocarbonyle, et
A représente le soufre,
ainsi que leurs sels de sodium, de potassium, de magnésium, de calcium, d'ammonium, de (alkyle en C₁ à C₄)ammonium, de di-(alkyle en C₁ à C₄)ammonium, de tri-(alkyle en C₁ à C₄)-ammonium, de tétra-(alkyle en C₁ à C₄)ammonium, de tri-(alkyle en C₁ à C₄)sulfonium, de (cycloalkyle en C₅ ou C₆)-ammonium et de di-(alkyle en C₁ ou C₂)benzylammonium.

2. Composés de formule (I) suivant la revendication 1, **caractérisé en ce que**, dans leur formule
R¹ est un groupe méthyle, éthyle, n-propyle, isopropyle, n-butyle, isobutyle, sec.-butyle ou tertio-butyle dont chacun est éventuellement substitué par du fluor, du chlore, un radical cyano, méthoxy ou éthoxy, un groupe propényle, butényle, propynyle ou butynyle dont chacun est éventuellement substitué par du fluor, du chlore ou du brome, un groupe méthoxy, éthoxy, n-propoxy, isopropoxy, n-butoxy, isobutoxy, sec.-butoxy, tertio-butoxy, propényloxy ou butényloxy dont chacun est éventuellement substitué par du fluor et/ou du chlore, un groupe méthylamino, éthylamino, n-propylamino, isopropylamino, n-butylamino, isobutylamino, sec.-butylamino, tertio-butylamino, diméthylamino ou diéthylamino, un groupe cyclopropyle, cyclobutyle, cyclopentyle, cyclohexyle, cyclopropylméthyle, cyclobutylméthyle, cyclopentylméthyle ou cyclohexylméthyle dont chacun est éventuellement substitué par du fluor, du chlore, du brome, un radical méthyle ou éthyle, ou bien un groupe benzyle ou phényle dont chacun est éventuellement substitué par du fluor, du chlore, du brome, un radical cyano, méthyle, trifluorométhyle ou méthoxy,
R² représente -CH₂-CF₃, -CH₂-CCl₃ ou -CH₂-CF₂-CHF₂ et
R³ représente le reste 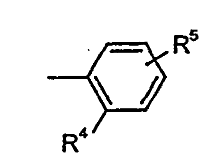 dans lequel
R⁴ représente le fluor, le chlore, le brome, un groupe méthyle, éthyle, n-propyle, isopropyle, trifluorométhyle, méthoxy, éthoxy, n-propoxy, isopropoxy, difluorométhoxy, trifluorométhoxy, 2-chloréthoxy, 2-méthoxyéthoxy, méthylthio, éthylthio, n-propylthio, isopropylthio, méthylsulfinyle, éthylsulfinyle, méthylsulfonyle, éthylsulfonyle, diméthylaminosulfonyle, diéthylaminosulfonyle, N-méthoxy-N-méthyl-aminosulfonyle, phényle, phénoxy, méthoxycarbonyle, éthoxycarbonyle, n-propoxycarbonyle ou isopropoxy-carbonyle, et
R⁵ représente l'hydrogène, le groupe méthyle, éthyle, le fluor, le chlore ou le brome ;
en outre
R³ représente le reste 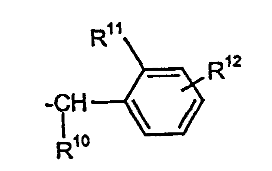 dans lequel
R¹⁰ est l'hydrogène,
R¹¹ représente le fluor, le chlore, le brome, un groupe méthyle, méthoxy, difluorométhoxy, trifluorométhoxy, éthoxy, méthoxycarbonyle, éthoxycarbonyle, méthylsulfonyle ou diméthylaminosulfonyle et
R¹² représente l'hydrogène ;
en outre
R³ représente le reste 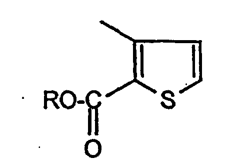 dans lequel
R est un groupe méthyle, éthyle, n-propyle ou isopropyle.

3. Procédé de production de composés de formule (I) suivant la revendication 1 et de leurs sels, **caractérisé en ce que** :
(a) on fait réagir des triazolinones de formule générale (II) 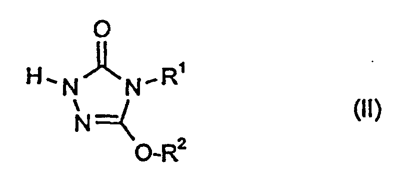 dans laquelle
R¹ et R² ont la définition indiquée dans la revendication 1,
avec des sulfonylisocyanates de formule générale (III)
R³-SO₂-N=C=O (III)
dans laquelle
R³ a la définition indiquée dans la revendication 1, le cas échéant en présence d'un auxiliaire de réaction et en présence éventuelle d'un diluant,
ou **en ce que** :
(b) on fait réagir des dérivés de triazolinones de formule générale (IV) 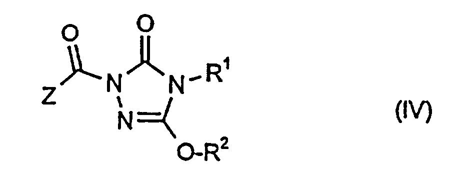 dans laquelle
R¹ et R² ont la définition indiquée ci-dessus et
Z représente un halogène, un groupe alkoxy, aralkoxy ou aryloxy,
avec des sulfonamides de formule générale (V)
R³-SO₂-NH₂ (V)
dans laquelle
R³ a la définition indiquée ci-dessus,
le cas échéant en présence d'un accepteur d'acide et en présence éventuelle d'un diluant,
ou **en ce que** :
(c) on fait réagir des triazolinones de formule générale (II) 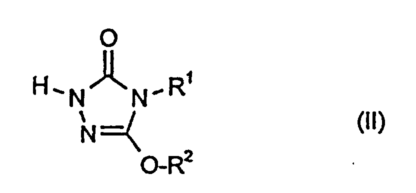 dans laquelle
R¹ et R² ont la définition indiquée ci-dessus,
avec des dérivés de sulfonamides de formule générale (VI)
R³-SO₂-NH-CO-Z (VI)
dans laquelle
R³ a la définition indiquée ci-dessus et
Z représente un halogène, un groupe alkoxy, aralkoxy ou aryloxy,
le cas échéant en présence d'un accepteur d'acide et en présence éventuelle d'un diluant,
ou **en ce que** :
(d) on fait réagir des triazolinones de formule générale (II) 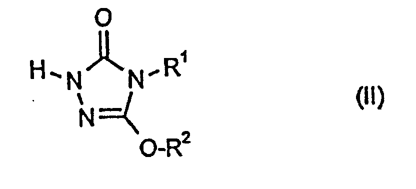
dans laquelle
R¹ et R² ont la définition indiquée ci-dessus,
avec des halogénures d'acides sulfoniques de formule générale (VII)
R³-SO₂-X (VII)
dans laquelle
R³ a la définition indiquée ci-dessus et
X représente un halogène,
et des cyanates métalliques de formule générale (VIII)
MOCN (VIII)
dans laquelle
M représente un métal alcalin ou un équivalent de métal alcalino-terreux,
le cas échéant en présence d'un auxiliaire de réaction et en présence éventuelle d'un diluant,
et on transforme éventuellement en sels par des modes opératoires classiques les composés de formule (I) obtenus selon les procédés (a), (b), (c) ou (d).

4. Compositions herbicides et fongicides, **caractérisées par** une teneur en au moins un composé de formule (I) suivant la revendication 1.

5. Utilisation de composés de formule générale (I) suivant la revendication 1 pour combattre une végétation indésirable et/ou des champignons phytopathogènes.

6. Procédé pour combattre des mauvaises herbes et/ou des champignons phytopathogènes, **caractérisé en ce qu'**on fait agir des composés de formule générale (I) suivant la revendication 1 sur les mauvaises herbes ou les champignons ou sur leur milieu.

7. Procédé de préparation de compositions herbicides ou fongicides, **caractérisé en ce qu'**on mélange des composés de formule générale (I) suivant la revendication 1 avec des diluants et/ou des agents tensio-actifs.

8. Triazolinones de formule générale (II) 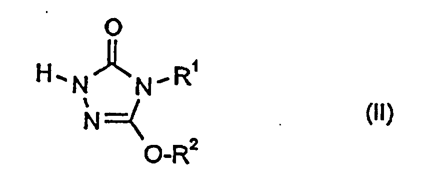 dans laquelle
R¹ et R² ont les définitions indiquées dans la revendication 1.

9. Dérivés de triazolinones de formule générale (IV) 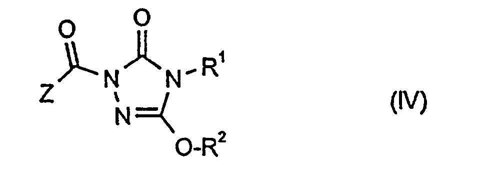 dans laquelle
R¹ et R² ont chacun les définitions indiquées dans la revendication 1, et
Z représente le fluor, le chlore, le brome, un groupe méthoxy, éthoxy, benzyloxy, phénoxy, halogénophénoxy ou nitrophénoxy.
